# EUROPEAN PATENT APPLICATION

(11) **EP 1 329 719 A1**
(43) Date of publication of application: **23.07.2003**
(21) Application number: 01972608.2
(22) Date of filing: 28.09.2001
(51) Int. Cl.: G01N 33/68, G01N 33/483

(54) **METHOD FOR ANALYZING ANTIBODY MOLECULE STRUCTURE**

(30) Priority: 29.09.2000 JP 2000299438
(71) Applicant: CHUGAI SEIYAKU KABUSHIKI KAISHA, Tokyo, 115-8543 (JP)
(72) Inventor: NISHIE, Itsuo c/o CHUGAI SEIYAKU KABUSHIKI KAISHA, Gotenba-shi, Shizuoka 412-8513 (JP)
(74) Representative: VOSSIUS & PARTNER
(86) International application number: JP0108577
(87) International publication number: WO02027328

(57) **Abstract**

The present invention provides a method for analyzing the primary structure of an antibody molecule by separating the H-chain and the L-chain of the antibody molecule, digesting each separated chain, and analyzing the peptide fragments obtained from each chain. The reproducibility can be elevated by combining in-gel digestion. According to the present method, an antibody molecule can be analyzed with a few steps and a high reproducibility. Moreover, quality of antibody molecules can be easily evaluated by the present method.

SEQUENCE LISTING

<110> CHUGAI SEIYAKU KABUSHIKI KAISYA

<120> Method for analyzing antibody molecule structure

<130> C1-A0012

<150> JP 2000-299438
<151> 2000-09-29

<160> 2

<170> PatentIn version 2.1

<210> 1
<211> 221
<212> PRT
<213> Homo sapiens

<220>
<221> MOD_RES
<222> (1)..(1)
<223> Pyroglutamic acid

<220>

<221>  MOD_RES
<222>  (22)..(22)
<223>  Carboxymethylcysteine

<220>
<221>  MOD_RES
<222>  (92)..(92)
<223>  Carboxymethylcysteine

<220>
<221>  MOD_RES
<222>  (143)..(143)
<223>  Carboxymethylcysteine

<220>
<221>  MOD_RES
<222>  (202)..(202)
<223>  Carboxymethylcysteine

<220>
<221>  MOD_RES
<222>  (220)..(220)
<223>  Carboxymethylcysteine

<400>  1
Xaa Leu Val Leu Thr Gln Ser Pro Ser Ala Ser Ala Ser Leu Gly Ala

1                   5                        10                       15
Ser Val Lys Leu Thr Xaa Thr Leu Ser Ser Gln His Ser Thr Tyr Thr
                20                       25                       30
Ile Glu Trp Tyr Gln Gln Gln Pro Glu Lys Gly Pro Lys Tyr Leu Met
            35                       40                       45
Asp Leu Lys Gln Asp Gly Ser His Ser Thr Gly Asp Gly Ile Pro Asp
    50                       55                       60
Arg Phe Ser Gly Ser Ser Ser Gly Ala Glu Arg Tyr Leu Thr Ile Ser
65                       70                       75                       80
Ser Leu Gln Ser Glu Asp Glu Ala Asp Tyr Ile Xaa Gly Val Gly Asp
                    85                       90                       95
Thr Ile Lys Glu Gln Phe Val Tyr Val Phe Gly Gly Gly Thr Lys Leu
                100                      105                      110
Thr Val Leu Gly Gln Pro Lys Ala Asn Pro Thr Val Thr Leu Phe Pro
            115                      120                      125
Pro Ser Ser Glu Glu Leu Gln Ala Asn Lys Ala Thr Leu Val Xaa Leu
    130                      135                      140
Ile Ser Asp Phe Tyr Pro Gly Ala Val Thr Val Ala Trp Lys Ala Asp
145                      150                      155                      160
Gly Ser Pro Val Lys Ala Gly Val Glu Thr Thr Lys Pro Ser Lys Gln
                    165                      170                      175
Ser Asn Asn Lys Tyr Ala Ala Ser Ser Tyr Leu Ser Leu Thr Pro Glu
                180                      185                      190
Gln Trp Lys Ser His Arg Ser Tyr Ser Xaa Gln Val Thr His Glu Gly
            195                      200                      205
Ser Thr Val Glu Lys Thr Val Ala Pro Thr Glu Xaa Ser

210                    215                    220

<210>  2

<211>  447

<212>  PRT

<213>  Homo sapiens

<220>

<221>  MOD_RES

<222>  (1)..(1)

<223>  Pyroglutamic acid

<220>

<221>  MOD_RES

<222>  (22)..(22)

<223>  Carboxymethylcysteine

<220>

<221>  MOD_RES

<222>  (96)..(96)

<223>  Carboxymethylcysteine

<220>

<221>  MOD_RES

<222>  (145)..(145)

<223>  Carboxymethylcysteine

<220>
<221>  MOD_RES
<222>  (201)..(201)
<223>  Carboxymethylcysteine

<220>
<221>  MOD_RES
<222>  (221)..(221)
<223>  Carboxymethylcysteine

<220>
<221>  MOD_RES
<222>  (227)..(227)
<223>  Carboxymethylcysteine

<220>
<221>  MOD_RES
<222>  (230)..(230)
<223>  Carboxymethylcysteine

<220>
<221>  MOD_RES
<222>  (262)..(262)
<223>  Carboxymethylcysteine

<220>

<221> MOD_RES

<222> (322)..(322)

<223> Carboxymethylcysteine

<220>

<221> MOD_RES

<222> (368)..(368)

<223> Carboxymethylcysteine

<220>

<221> MOD_RES

<222> (426)..(426)

<223> Carboxymethylcysteine

<400> 2

Xaa Val Gln Leu Val Glu Ser Gly Gly Gly Val Val Gln Pro Gly Arg

1               5                   10                  15

Ser Leu Arg Leu Ser Xaa Ala Ala Ser Gly Phe Thr Phe Ser Ser Tyr

            20                  25                  30

Gly Met Ser Trp Val Arg Gln Ala Pro Gly Lys Gly Leu Glu Trp Val

        35                  40                  45

Ala Thr Ile Ser Ser Gly Gly Ser Tyr Thr Tyr Tyr Pro Asp Ser Val

    50                  55                  60

Lys Gly Arg Phe Thr Ile Ser Arg Asp Asn Ser Lys Asn Thr Leu Tyr

65                  70                  75                  80
Leu Gln Met Asn Ser Leu Arg Ala Glu Asp Thr Ala Val Tyr Tyr Xaa
                85                  90                  95
Ala Arg Gln Thr Thr Met Thr Tyr Phe Ala Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Xaa Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Xaa Asn Val Asn His Lys Pro Ser
        195                 200                 205
Asn Thr Lys Val Asp Lys Lys Val Glu Pro Lys Ser Xaa Asp Lys Thr
    210                 215                 220
His Thr Xaa Pro Pro Xaa Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Xaa Val Val Val Asp Val Ser His Glu Asp Pro
            260                 265                 270
Glu Val Lys Phe Asn Trp Tyr Val Asp Gly Val Glu Val His Asn Ala

        275                 280                 285

Lys Thr Lys Pro Arg Glu Glu Gln Tyr Asn Ser Thr Tyr Arg Val Val

    290                 295                 300

Ser Val Leu Thr Val Leu His Gln Asp Trp Leu Asn Gly Lys Glu Tyr

305                 310                 315                 320

Lys Xaa Lys Val Ser Asn Lys Ala Leu Pro Ala Pro Ile Glu Lys Thr

                325                 330                 335

Ile Ser Lys Ala Lys Gly Gln Pro Arg Glu Pro Gln Val Tyr Thr Leu

            340                 345                 350

Pro Pro Ser Arg Asp Glu Leu Thr Lys Asn Gln Val Ser Leu Thr Xaa

        355                 360                 365

Leu Val Lys Gly Phe Tyr Pro Ser Asp Ile Ala Val Glu Trp Glu Ser

    370                 375                 380

Asn Gly Gln Pro Glu Asn Asn Tyr Lys Thr Thr Pro Pro Val Leu Asp

385                 390                 395                 400

Ser Asp Gly Ser Phe Phe Leu Tyr Ser Lys Leu Thr Val Asp Lys Ser

                405                 410                 415

Arg Trp Gln Gln Gly Asn Val Phe Ser Xaa Ser Val Met His Glu Ala

            420                 425                 430

Leu His Asn His Tyr Thr Gln Lys Ser Leu Ser Leu Ser Pro Gly

        435                 440                 445

## Description

### Technical Field

The present invention relates to a method for analyzing the primary structure of an antibody molecule. More specifically, the present invention relates to a method for analyzing the primary structure of an antibody molecule by separating the H-chains and the L-chains of the antibody molecule, fragmenting each separated chain, and analyzing the peptide fragments obtained from each chain.

Moreover, this invention provides a method for producing peptide maps for the H-chains and the L-chains of an antibody molecule, respectively, by analyzing the peptide fragments obtained by digestion. In addition, this invention provides a method for estimating the subclass of the H-chain and the type of the L-chain of a structurally unidentified antibody.

### Background Art

An antibody molecule consists of H-chains and L-chains. For example, IgG is composed of a tetramer wherein two H-chains and two L-chains are linked via disulfide bonds (S-S bonds). Several structural heterogeneities may occur among antibody molecules. Commonly reported examples include N-terminal blocking, C-terminal amino acid deletion, deamidation, oxidation, methylation, succinylation, and glycosylation. Because these structural heterogeneities influence the function and stability of antibodies, identification of heterogeneity is important. Particularly, when utilizing antibody molecules as pharmaceuticals, analysis of heterogeneity becomes important for quality evaluation. Furthermore, confirmation of the amino acid sequence in a preliminary evaluation of physical properties, and construction of a peptide mapping system for a drug substance standardization test and quality assurance are mandatory for advancing drug development. Therefore, an analysis system that enables more convenient and more rapid examination becomes necessary.

In primary structure analysis of antibody molecules, fragmentation is performed by enzymatic or chemical methods. However, antibody molecules generally have multimeric structures, and also have high molecular weights. For example, the molecular weight of IgG, a representative antibody molecule, is approximately 150,000 daltons. Therefore, direct fragmentation of an antibody molecule produces a complex mixture of peptide fragments, which makes the separation and assignment of each fragment difficult. Thus, high reproducibility cannot be achieved according to such a method.

To avoid the influence of an antibody being a multimer, fragmentation after separation into H-chains and L-chains by liquid chromatography, such as reverse phase high performance liquid chromatography (RP-HPLC), can be considered. However, besides S-S bonds (covalent bonds), H-chains and L-chains are strongly bound by non-covalent bonds such as ionic bonds, hydrophobic bonds, and hydrogen bonds; thus, fractionation of H-chains and L-chains by liquid chromatography with good recovery is difficult. Therefore, in conventional methods for analysis, large amounts of antibodies are needed as samples.

### Disclosure of the Invention

An objective of this invention is to provide a method for recovering peptide fragments that are obtained by fragmenting an antibody molecule in good yield, in terms of both the kind and the amount of the fragment, and analyzing them for primary structure analysis of the antibody molecule. In addition, another objective of the present invention is to provide a method for more conveniently and more quickly analyzing heterogeneity and evaluating the quality of antibody molecules by separating and fractionating peptide fragments that were obtained by fragmenting an antibody molecule via appropriate separation techniques (for example RP-HPLC), then constructing a peptide map having a good reproducibility based on the results of analysis. Furthermore, this invention provides a method for efficiently analyzing the variable regions by readily identifying the subclass of the H-chain and the type of the L-chain of a structurally unidentified antibody via a comparison of the obtained peptide maps.

To accomplish the aforementioned objective, the present inventors focused on the structural characteristics of antibody molecules. As a result, they found out that besides the large molecular weight of an antibody molecule, the multimeric structure thereof is an obstacle in analyzing the antibody molecule. In addition, they demonstrated that the aforementioned obstacles can be overcome by separating in advance an antibody molecule into its H-chains and L-chains, and fragmenting the chains in the separated state. This invention was accomplished based on these findings. That is, the present invention relates to the following method of analysis, and use of the same.
[1] A method for structural analysis of an antibody molecule, which comprises the steps of:
   a) separating the antibody molecule into H-chains and L-chains;
   b) fragmenting the separated H-chains and L-chains; and
   c) analyzing the peptide fragments obtained by fragmentation.
[2] The method of [1], wherein the antibody molecule is separated into H-chains and L-chains by denaturing gel electrophoresis.
[3] The method of [1], wherein disulfide bonds are cleaved by reduction prior to separating the antibody molecule into H-chains and L-chains.
[4] The method of [3], wherein the thiol groups formed by reducing the disulfide bonds of the antibody molecule are made irreversible by modification.
[5] The method of [2], wherein the separated H-chains and L-chains are fragmented in gel.
[6] The method of [5] which comprises the step of eluting the fragmented peptides from the gel.
[7] The method of [2], wherein the separated H-chains and L-chains are transferred to a protein adsorption membrane, and then are fragmented on the protein adsorption membrane.
[8] The method of [1] comprising the step of separating the peptide fragments obtained by fragmentation.
[9] The method of [8] additionally comprising the step of deblocking peptide fragments whose N-termini are blocked.
[10] The method of [1], wherein the analysis of peptide fragments is performed by amino acid sequence analysis.
[11] The method of [1], wherein the analysis of peptide fragments is performed by mass spectrometry.
[12] The method of [1], wherein the analysis of peptide fragments is performed by amino acid composition analysis.
[13] A peptide map which is produced based on results of structural analysis obtainable by the method of [1].
[14] The method of [1] wherein the type of heterogeneity is determined by analyzing peptide fragments involved in heterogenity among the peptide fragments obtained by separation.
[15] The method of [1], wherein the antibody is an antibody for pharmaceutical use.
[16] A method of evaluating the quality of an antibody molecule, which comprises the steps of:
   a) separating the antibody molecule into H-chains and L-chains;
   b) fragmenting the separated H-chains and L-chains;
   c) analyzing the peptide fragments obtained by fragmentation;
   d) comparing the obtained analysis result with an analysis result obtained by analyzing a structurally identified antibody; and
   e) determining that the two antibodies are the same when there is no difference between the two analysis results, or when the difference is within a specified range.
[17] A method for identifying the subclass of the H-chain and/or the type of the L-chain of a structurally unidentified antibody by analyzing the structures of a structurally identified antibody and a structurally unidentified antibody, respectively, by the method of [1], and then comparing the analysis results relating to both constant regions.
[18] A method for analyzing the variable region of an antibody molecule, which comprises the steps of:
   a) analyzing the structure of the antibody molecule by the method of [1]; and
   b) selecting information on the variable region by removing information on the constant region from the analyzed information of a) based on the analysis result of a structurally identified antibody.
[19] The method of [18], wherein the analysis method is reverse-phase liquid chromatography, and step b) involves selecting peaks corresponding to the variable region from peaks corresponding to the constant region.

In this invention, first, the antibody molecule is separated into H-chains and L-chains to facilitate primary structure analysis of the antibody molecule. Next, both of the separated chains are fragmented independently, and the obtained peptide fragments are analyzed. Based on the results of analysis of the obtained peptide fragments, a peptide map can be prepared. The peptide map can be used to evaluate the quality of the antibody molecule, and such.

In the present invention, the H-chains and L-chains of an antibody molecule can be separated by any method. For example, the antibody molecule can be cleaved at the S-S bond by a treatment with reducing agent, such as dithiothreitol (DTT) and 2-mercaptoethanol (2-ME), and then, separated into the H-chains and L-chains based on a difference in molecular weight. By modifying the thiol group (SH group) after reductive treatment, recombination of the S-S bond can be prevented. S-S bonds are not only formed between the originally bonded SH groups, but also between completely unrelated SH groups. Therefore, recombination of S-S bonds results in the formation of a complexed mixture.

SH groups can be modified by alkylation, acylation, and such. More specifically, treatments such as reduction and carboxymethylation, reduction and pyridylethylation, and reduction and carboxyamidemethylation can be presented as examples. The S-S bond becomes irreversible by modifying the SH group, and thus production of a complexed mixture due to recombination is suppressed. Therefore, modification of the SH group is expected to increase the accuracy of the analysis.

In the present invention, modification of the SH group can be performed at any time, e.g., before separation of the H-chains and L-chains, in the gel after separation but before fragmentation, or after fragmentation. Particularly, separation by SDS-PAGE, as mentioned in Examples, can be accomplished with good yield and also at a high reproducibility by performing the modification before the separation. Specifically, the separation by SDS-PAGE is performed after denaturation by sodium dodecyl sulfate (SDS) , reduction by DTT, and carboxymethylation treatment.

In this invention, antibody molecules cleaved at S-S bonds are separated into H-chains and L-chains prior to fragmentation. H-chains and L-chains can be separated by any method. H-chains and L-chains can be separated based on the differences in their properties (for example, molecular weight, electric charge, hydrophobicity). Specifically, separation techniques such as electrophoresis and liquid chromatography can be used. However, the separation must be conducted under conditions that hinder recombination of the H-chains and L-chains. More specifically, it is advantageous to separate the H-chains and L-chains by denaturing gel electrophoresis. By employing denaturing gel electrophoresis, the H-chains and L-chains can be physically separated, and a higher reproducibility can be expected.

In the present invention, the term "denaturing gel electrophoresis" refers to gel electrophoresis that enables separation of the H-chains and L-chains of antibody molecules. Electrophoresis which utilizes an acrylamide gel medium containing SDS and urea is well known as an example for such electrophoresis. Particularly, SDS-PAGE is an especially advantageous separation technique for use in this invention. SDS-PAGE can be performed according to the method of Laemmli (Laemmli (1970) Nature 227, 680-685). The H-chains and L-chains are strongly bound by covalent bonds (S-S bonds) as well as non-covalent bonds, but by using SDS-PAGE, the H-chains and L-chains can be readily separated.

Alternatively, denaturing gel electrophoresis can be also carried out without performing reductive acylation and such. For example, when only information of a part of the internal sequence of an antibody molecule is required, H-chains and L-chains can be separated even by SDS-PAGE under reducing conditions, and the primary structure can be analyzed in a similar manner. Therefore, the amino acid sequence without the cysteine residues can be investigated.

Each of the separated H-chains and L-chains are fragmented. Fragmentation methods for primary structure analyses of proteins are well known. Generally, for structural analyses of proteins, enzymatic digestion and chemical cleavage are utilized. For example, the following enzymes may be used. All of these enzymes are commercially available, and their digestion conditions are known.
Lysyl endopeptidase
Endoproteinase LysC
Mouse submandibular gland protease D
*Staphylococcus aureuse* V8 protease
Endoproteinase Asp-N

Protein digestion by enzymatic digestion can be performed under a mild condition, and therefore, modifications and such of the side chains of amino acid residues are unlikely to happen. Furthermore, by fixing the conditions for digestion, reproducibility can be easily maintained.

In this invention, chemical cleavage can be utilized instead of enzymatic digestion. Cleavage at the Carboxyl terminus of a methionine residue by cyanogen bromide is a representative chemical cleavage method.

Fragmentation of the separated H-chains and L-chains can be performed by any method. For example, as a technique recently developed for structural analysis of proteins, there is a method wherein bands or spots that are obtained by subjecting a protein to SDS-PAGE or two-dimensional electrophoresis are excised, and then chemically or enzymatically fragmented in a gel (Jahnen-Dechent and Simpson (1990) Plant Mol. Biol. Reporter 8, 92-103; Hellman U. et al., (1995) Anal. Biochem. 224, 451-455; Kawasaki et al. (1990) Anal. Biochem. 191, 332-336). According to this method, proteins are fragmented in a gel. Therefore, the method is called an in-gel digestion method. Alternatively, proteins separated in a gel can be fragmented on membranes, such as polyvinylidene difluoride (PVDF) membrane and nitrocellulose membrane, after transferring the separated protein on the membrane. In this invention, the reproducibility of the analysis can be further raised by combining the in-gel digestion method or the fragmentation method on a protein adsorption membrane to the aforementioned fragmentation process. PVDF membranes are commercially available under product names such as "Immobilon" (MILLIPORE).

The in-gel digestion method, one of the analysis methods of proteome analysis, is normally carried out by the steps of enzymatically digesting a protein separated and purified by two-dimensional electrophoresis directly in a gel, and then obtaining information on its amino acid sequence from the recovered peptide fragments. In this invention, an antibody can be easily separated into H-chains and L-chains by denaturing gel electrophoresis, such as SDS-PAGE. Therefore, the inventors anticipated that fragmentation for the present invention could be accomplished by restricting electrophoresis to denaturing gel electrophoresis and performing enzymatic digestion of the separated H-chains and L-chains directly in the gel. That is, the only electrophoresis performed prior to enzymatic digestion is, not two-dimensional electrophoresis, but denaturing gel electrophoresis.

The in-gel digestion method can be performed by excising the bands of H-chains and L-chains separated by SDS-PAGE; incubating them after adding a digestion buffer to soak the gel; and then adding a protease, such as Lys-C, for digestion. The digest solution is recovered, eluting buffer is freshly added to the gel, and the fragments are extracted from the gel. This operation is repeated, and the obtained digest solution and eluate are mixed for following structural analysis. According to the in-gel digestion method, H-chains and L-chains can be readily fragmented in few steps, and the subsequent separation and fractionation of the peptide fragments can be performed with a good recovery. Alternatively, in this invention, the H-chains and L-chains separated by SDS-PAGE can be transferred onto a PVDF membrane to fragment them onto the PVDF membrane. Transfer to a PVDF membrane and fragmentation thereon can be performed according to conventional methods.

The three-dimensional structure of an antibody molecule is supported by diverse bonding patterns, including S-S bonds. Therefore, for example, when only the S-S bonds are dissociated and other bonds cannot be sufficiently blocked, various three-dimensional structures are formed during digestion. Construction of such three-dimensional structures is one of the factors that prevent fragmentation of the peptides. In the analysis method of this invention, due to the restricted movement of separated H-chains and L-chains, obtained by combining the in-gel digestion method or the fragmentation method with a protein adsorption membrane, digestion of the fragments can be carried out under conditions wherein the construction of other three-dimensional structure is greatly suppressed. As a result, analysis can be performed with a good reproducibility.

The present invention can be utilized to analyze any kind of antibody. According to the present invention, structural analyses can be conducted for. naturally occurring antibodies, chimeric antibodies and human antibodies, as well as derivatives or modified derivatives thereof. The present invention can not only be applied to complete antibody molecules (whole antibodies) but also to antibody fragments, such as Fab, F(ab')2, and Fab'. Furthermore, the method of this invention can not only be applied to IgG but also to all classes of antibodies, such as IgA, IgM, IgD, and IgE. Moreover, there are no restrictions with regard to the particular animal specie or cell line from which the antibody to be analyzed is derived. In addition, antibodies prepared by any method can be analyzed by the method of the present invention, so long as the antibody molecules contain H-chains and L-chains that are separable.

Peptide fragments obtained by the digestion of H-chains and L-chains of an antibody molecule can be analyzed according to known protein analysis methods. In this invention, the analysis of a peptide fragment is defined as revealing the biochemical information possessed by the peptide fragment. Examples of biochemical information include information regarding amino acid sequence of the peptide fragment, molecular weight, mass, presence or absence and type of modification, and so on. Modifications of peptide fragments include deamidation, oxidation, methylation, succinylation, glycosylation, and so on. If necessary, the peptide fragments obtained by digestion may be analyzed after separating them by appropriate techniques. Such techniques include RP-HPLC, SDS-PAGE, ion-exchange chromatography, and such. Separation patterns of a peptide fragment obtained by the above-mentioned separation technique can be used as a peptide map. Furthermore, regarding peptide fragments related to heterogeneity, the type of heterogeneity can be determined by applying appropriate analysis methods. In this invention, since the heterogeneity existing in the antibody molecule is analyzed after fragmentation of both of the H-chains and L-chains, analysis can be performed readily. Methods for analysis are well known in the art.

General methods for analyzing peptide fragments include mass spectrometry, amino acid sequence analysis, amino acid composition analysis, and so on. Each of these methods for structural analysis can be performed according to conventional methods. Mass spectrometry is the most reliable analysis method at present among analysis methods based on the difference in molecular weight. In the present invention as well, mass spectrometry is one of the preferred analysis methods. A mass spectrometer is composed of an ion source, a mass detection element, and a detector. Various methods for ionization and various methods for a detection piece are known, and instruments are composed of combinations of part utilizing these methods. Representative methods are shown below.
Ionization methods-
MALDI (Matrix Assisted Laser Desorption Ionization)
EI (Electron Ionization)
FAB (Fast Atom Bombardment)
API (Atmosphere Pressure Ionization)
Detection methods-
TOF (Time of Flight)
Sector (Magnetic, Electrostatic)
Quadrupole
FT (Fourier Transform)

Among the combinations, MALDI-TOF-MS (TOF-MS) is an especially advantageous analysis instrument for the present invention. There also are instruments, such as LC-MS wherein liquid chromatography (LC) is combined with mass spectrometry, tandem MS (MS/MS) wherein two mass spectrometers are connected together, and LC-MS/MS wherein the LC and MS/MS are combined; and each of them are used depending on the objective of the analysis.

Amino acid sequence is the most important data in the primary structure analysis of a protein. In this invention as well, identification of peptide fragments is based on the result of sequence analysis. Representative methods for amino acid sequence analysis are shown below.
N-terminus analysis methods-
Edman degredation method (PITC method)
DABITC method
Dansyl method (DNS-Cl method)
Aminopeptidase method
C-terminus analysis methods-
Hydrazinolysis method
Carboxypeptidase method

At present, an automatic analyzer using the Edman degradation method exists and analysis using this instrument is generally performed. The instrument was also used in the examples of this invention discussed in detail below. In the Edman degradation method, blocking of the N-terminus of the peptide fragment becomes an obstacle. In this invention, amino acid sequence analysis can be performed by the Edman degradation method after deblocking the N-terminus. Specifically, deblocking can be easily and reliably performed by conducting the deblocking step after the fragmentation of the peptide. Methods for deblocking the N-terminus are well known in the art.

Blocking of the N-terminus refers to a condition wherein the α-amino group of the N-terminal amino acid is modified by acetylation and such. It is difficult to identify amino acids whose α-amino group is modified by methods such as the Edman degradation method. However, the blocked N-terminus can be analyzed by removing the modification. For example, the modification of an acetylated amino acid can be enzymatically removed with an amino acid acylase. Generally, the amino acid acylase acts poorly on large proteins. In this invention, the amino acid acylase will be made to act on digested fragments. Therefore, in this invention, the N-terminus can be easily and reliably deblocked.

Amino acid composition analysis is necessary to understand the basic characteristics of a protein. Conventionally, the amino acid composition of a protein is detected by derivatization of the amino acids composing the protein that are formed due to hydrolysis. The detection methods are categorized into two types, depending on whether the derivatization is carried out before or after the amino acid separation. Representative reagents for derivatization are shown below.
Post-column methods-
ninhydrin
*o*-phthalaldehyde
Pre-column methods-
PITC
NBD-F
FMO-Cl
Dabsyl-Cl
*o*-phthalaldehyde
DNS-Cl

The post-column method using ninhydrin has been automated and has a high reproducibility. However, due to the problem of detection limit, pre-column method using PITC is often utilized for the primary structure analysis of proteins.

Furthermore, the present invention relates to peptide maps that can be obtained by the analysis methods of this invention. Based on such analysis results, peptide maps relating to the structure of antibody molecules can be prepared. In this invention, the term "peptide map" refers to a set of information of each peptide fragment that compose a certain peptide mixture, which can characterize the peptide mixture. The term "peptide map" generally refers to a condition wherein a mixture of peptide fragments has been separated by some method. However, herein, not only separated peptide fragments themselves but also a set of information obtained by analyzing the peptide fragments are referred to as the peptide map. Therefore, electrophoretic image developed by electrophoresis of digested fragments is included in the peptide map of this invention. Furthermore, the results of mass spectrometry of peptide fragments are also included in the peptide map of this invention.

Structural equivalence or difference in original proteins can be determined by comparing peptide maps in order to compare the characteristic of a certain peptide fragment mixture to that of another peptide fragment mixture. A high possibility that the original antibodies share a common structure is suggested by peptide fragment mixtures yielding common peptide maps. On the contrary, difference in the peptide maps indicate structural differences of the original antibody molecules. Therefore, antibody molecule structures can be compared by comparing peptide maps.

For example, a quality of an antibody can be evaluated by comparing the analysis result with that of a structurally identified antibody. More specifically, a peptide map of a produced antibody molecule is prepared according to the present invention, and is compared with a peptide map that is used as the standard. As a result, when the difference between both peptide maps is within the range determined based on the desired precision of analysis, the produced antibody is regarded as equivalent, including its heterogeneity. In the present invention, heterogeneity refers to structural differences among antibody molecules. Structural differences of antibody molecules are caused, for example, by the presence of N-terminal blocking, deletion of the C-terminal amino acid, deamidation, oxidation, methylation, succinylation, and glycosylation. However, a heterogeneity that can be discovered according to the present invention is not limited to those caused by the factors illustrated herein.

Using a peptide map prepared based on the method of this invention, the subclass and type of a structurally unidentified antibody can be identified. Specifically, the subclass of the H-chain and the type of L-chain of a structurally unidentified antibody can be identified by preparing peptide maps based on the method of the present invention for a structurally identified antibody and the structurally unidentified antibody, respectively; and comparing the constant regions of the two. Furthermore, from a mixture of multiple structurally unidentified antibodies, the subclasses and types the mixture is composed can be easily determined by comparing its peptide map with peptide maps prepared for structurally identified antibodies of different subclasses and types.

An immunoglobulin is composed of constant regions and variable regions. The variable regions have various structures due to antigen recognition, whereas the structure of the constant regions is limited. Therefore, peptide maps for all expected constant regions can be prepared in advance. The structure of the constant region is categorized into the subclasses of the H-chains and into the types of the L-chains. When peptide maps are prepared for all the subclasses and types, the subclass of the H-chain or the type of the L-chain composing a structurally unidentified immunoglobulin can be determined by preparing its peptide map by the same method, and comparing the peptide map to the known peptide maps.

By utilizing peptide map information on fragments derived from the constant region, primary structure analysis of an unidentified antibody can be efficiently carried out. As already mentioned above, an antibody molecule consists of a constant region and a variable region. Therefore, to obtain information on fragments derived from the constant region from a peptide map is nothing but to predict peaks of fragments derived from the variable region. Thus, the structure of fragment peaks estimated to stem from the variable region can be preferentially analyzed. In the evaluation of antibody molecules, structural information on the variable region that affects the binding specificity with an antigen is important. Thus, the technique of the present invention that enables one to efficiently proceed to the analysis of the variable region is useful as a method for evaluating antibody molecules.

For example, as indicated in Examples below, when the analysis of peptide fragments are performed by reverse-phase chromatography and mass spectrometry, then the peptide map is obtained as a chromatogram or a mass spectrum. Evaluation of an antibody molecule by comparing peptide maps is carried out via the decision of peak assignment and such. With regard to the evaluation, once the peaks of the fragments derived from the constant region are identified, the variable region can be analyzed by comparing the remaining peaks.

Furthermore, the amino acid sequence of fragments that are derived from the variable region can be analyzed by selecting peaks of the fragments in a chromatogram of a reverse-phase chromatography, and fractionating the selected peaks. According to the present invention, structural analysis of variable region can efficiently conducted in this manner.

### Brief Description of the Drawings

Fig. 1 shows the result of analysis of a mixture of Lys-C digests of humanized PTHrP antibody L-chain by TOF-MS. The numbers at the peaks indicate the measured mass and the fragment number presumed to correspond thereto.
Fig. 2 shows the result of analysis of a mixture of Lys-C digests of humanized PTHrP antibody H-chain by TOF-MS. The numbers at the peak tops indicate the measured mass and the fragment number presumed to correspond thereto.
Fig. 3 shows the chromatogram wherein a mixture of Lys-C digests of humanized PTHrP antibody L chain was fractionated by RP-HPLC. The fractions were analyzed with a sequencer and fragment numbers assigned thereto are indicated at the peak tops.
Fig. 4 shows the chromatogram wherein a mixture of Lys-C digests of humanized PTHrP antibody H chain was fractionated by RP-HPLC. The fractions were analyzed with a sequencer and fragment numbers assigned thereto are indicated at the peak tops.
Fig. 5 shows the result of TOF-MS of an RP-HPLC fraction corresponding to the fragment CH17-18 that comprises the residue 298 of the H-chain of humanized PTHrP antibody.
Fig. 6 shows the comparison of peptide maps for the L-chains of humanized PTHrP antibody, humanized anti-HM1.24 antibody, and humanized anti-TF antibody, respectively, that were obtained according to the method of the present invention using Lys-C as the digestive enzyme. The peaks of fragments corresponding to the constant regions are connected with straight lines. The results of humanized PTHrP antibody, humanized anti-HM1.24 antibody, and humanized anti-TF antibody are shown in the figure in order from the top.
Fig. 7 shows the comparison of peptide maps for the H-chains of humanized PTHrP antibody, humanized anti-HM1.24 antibody, and humanized anti-TF antibody, respectively, that were obtained according to the method of the present invention using Lys-C as the digestive enzyme. The peaks of fragments corresponding to the constant region are connected with straight lines. The results of humanized PTHrP antibody, humanized anti-HM1.24 antibody, and
humanized anti-TF antibody are shown in the figure in order from the top.
Fig. 8 shows a peptide map for humanized PTHrP antibody that was prepared using Lys-C without reduction and carboxymethylation. It shows that a peptide map can be prepared without reduction and carboxymethylation.
Fig. 9 shows the result of analysis on a mixture of Asp-N digests of humanized PTHrP antibody L-chain by TOF-MS. The fragment numbers and the theoretical mass (in parenthesis) are shown at the peak tops.
Fig. 10 shows the result of analysis on a mixture of Asp-N digests of humanized PTHrP antibody H-chain by TOF-MS. The fragment numbers and the theoretical mass (in parenthesis) are shown at the peak tops.
Fig. 11 shows the chromatogram wherein a mixture of Asp-N digests of humanized PTHrP antibody L chain was fractionated by RP-HPLC. The fractions were analyzed with a sequencer and the fragment numbers assigned thereto are indicated at the peak tops.
Fig. 12 shows the chromatogram wherein a mixture of Asp-N digests of humanized IL-6R antibody L chain was fractionated by RP-HPLC. The fractions were analyzed with a sequencer and the fragment numbers assigned thereto are indicated at the peak tops.
Fig. 13 shows the comparison of peptide maps for the L-chains of humanized PTHrP antibody, humanized anti-IL-6R antibody, and humanized anti-TF antibody, respectively, that were obtained according to the method of the present invention using Asp-N as the digestive enzyme. The peaks of fragments corresponding to the constant region are shown connected with straight lines. The results of humanized PTHrP antibody, humanized IL-6R antibody, and humanized anti-TF antibody are shown in the figure in order from the top.
Fig. 14 shows the comparison of peptide maps for the H-chains of humanized PTHrP antibody, humanized anti-IL-6R antibody, and humanized anti-TF antibody, respectively, that were obtained according to the method of the present invention using Asp-N as the digestive enzyme. The peaks of fragments having the same amino acid sequence are connected with straight lines. The results of humanized PTHrP antibody, humanized IL-6R antibody, and humanized anti-TF antibody are shown in the figure in order from the top.
Fig. 15 shows the chromatograms wherein different sample volumes of a mixture of the Asp-N digest of humanized PTHrP antibody L chain were fractionated by RP-HPLC. The results for sample volumes of 30, 10, 5, 2, and 1 µg are shown in order from the top.
Fig. 16 shows the result of analyzing a mixture of the Lys-C digest of humanized HM1.24 antibody H-chain by TOF-MS. The fragment numbers are indicated at the peak tops for the assigned peaks.
Fig. 17 shows the result of analyzing a mixture of the Lys-C digest of humanized HM1.24 antibody L-chain by TOF-MS. The fragment numbers are indicated at the peak tops for the assigned peaks.
Fig. 18 shows the result of analyzing a mixture of the Lys-C digest of humanized TF antibody H-chain by TOF-MS. The fragment numbers are indicated at the peak tops for the assigned peaks.
Fig. 19 shows the result of analyzing a mixture of the Lys-C digest of humanized TF antibody L-chain by TOF-MS. The fragment numbers are indicated at the peak tops for the assigned peaks.
Fig. 20 shows the result of analyzing a mixture of the Lys-C digest of humanized IL-6R antibody H-chain by TOF-MS. The fragment numbers are indicated at the peak tops for the assigned peaks.
Fig. 21 shows the result of analyzing a mixture of the Lys-C digest of humanized IL-6R antibody L-chain by TOF-MS. The fragment numbers are indicated at the peak tops for the assigned peaks.
Fig. 22 shows the comparison of peptide maps for the L-chains of humanized PTHrP antibody, humanized anti-HM1.24 antibody, humanized anti-TF antibody, and humanized IL-6R antibody, respectively, that were obtained according to the method of the present invention by TOF-MS using Lys-C as the digestive enzyme. The peaks of fragments corresponding to the constant regions are connected with straight lines. The results of humanized PTHrP antibody, humanized anti-HM1.24 antibody, humanized anti-TF antibody, and humanized IL-6R antibody are shown in the figure in order from the top.
Fig. 23 shows the comparison of peptide maps for the H-chains of humanized PTHrP antibody, humanized anti-HM1.24 antibody, humanized anti-TF antibody, and humanized IL-6R antibody, respectively, that were obtained according to the method of the present invention by TOF-MS using Lys-C as the digestive enzyme. The peaks of fragments corresponding to the constant regions are connected with straight lines. The results of humanized PTHrP antibody, humanized anti-HM1.24 antibody, humanized anti-TF antibody, and humanized IL-6R antibody are shown in the figure in order from the top.
Fig. 24 shows the result of analysis on a mixture of the Lys-C digest of humanized HM1.24 antibody H chain by RP-HPLC. The fragment numbers are indicated at the peak tops for the assigned peaks.
Fig. 25 shows the result of analysis on a mixture of the Lys-C digest of humanized HM1.24 antibody L chain by RP-HPLC. The fragment numbers are indicated at the peak tops for the assigned peaks.
Fig. 26 shows the result of analysis on a mixture of the Lys-C digest of humanized TF antibody H chain by RP-HPLC. The fragment numbers are indicated at the peak tops for the assigned peaks.
Fig. 27 shows the result of analysis on a mixture of the Lys-C digest of humanized TF antibody L chain by RP-HPLC. The fragment numbers are indicated at the peak tops for the assigned peaks.
Fig. 28 shows the result of analysis on a mixture of the Lys-C digest of humanized IL-6R antibody H chain by RP-HPLC. The fragment numbers are indicated at the peak tops for the assigned peaks.
Fig. 29 shows the result of analysis on a mixture of the Lys-C digest of humanized IL-6R antibody L chain by RP-HPLC. The fragment numbers are indicated at the peak tops for the assigned peaks.
Fig. 30 shows the result of analysis on a mixture of the Asp-N digest of humanized PTHrP antibody H chain by RP-HPLC. The fragment numbers are indicated at the peak tops for the assigned peaks.

### Best Mode for Carrying out the Invention

The present invention will be explained in detail below with reference to Examples.

### [Example 1]

Hereinafter, an example of analysis according to the present invention will be described using an anti-Parathyroid hormone-related peptide humanized antibody (IgG1) (method for preparing the antibody is described in WO98/13388) as the example.

The amino acid sequences of humanized PTHrP antibody L-chain and H-chain are shown in SEQ ID NOs: 1 and 2, respectively. The humanized PTHrP antibody is an IgG1 antibody which has a structure wherein two each of H-chains and L-chains are cross-linked by S-S bonds; it is a huge protein, consisting of round about 1,300 amino acids in total. Therefore, when the entire antibody molecule was analyzed at once, reproducibility was difficult to maintain regarding two points: enzymatic digestion, separation and fractionation of the fragments. Furthermore, even when the S-S bonds were irreversibly cleaved by reductive modification, the H-chains and L-chains were separated by RP-HPLC, and then were analyzed respectively, the separation of the H-chains and L-chains was insufficient, recovery was low, and precision of analysis was not good. Since an antibody is a huge protein, the situation was such that a highly accurate data could not be obtained by just applying conventional methods for protein analysis. Therefore, the method of this invention was developed.

### (Procedure)

### 1) Sample preparation

### a) Reduction and carboxymethylation

Humanized PTHrP antibody solution (13.4 mg protein/ml; 150 mM NaCl, 20 mM acetic acid/sodium acetate buffer, pH 5.7) and SDS sample buffer (5% SDS, 2 mM ethylenediamine tetraacetic acid (EDTA), 0.005% bromophenol blue (BPB), 20 mM tris(hydroxymethyl)aminomethane (Tris)-HCl, pH 8.0) were mixed at a ratio of 1:5 (v/v) while stirring, and denaturation was carried out by heating at 95°C in a water bath for 5 minutes. Reduction was carried out by adding DTT to a final concentration of 40 mM, substituting the upper space in the tube with nitrogen gas, and then incubating in a thermoregulated air bath at 37°C for 2 hours. Carboxymethylation was carried out by adding iodoacetic acid to a final concentration of 80 mM, purging the upper space in the tube with nitrogen gas, and performing the reaction in a thermoregulated air bath at 37°C for 30 minutes. The mixture was neutralized to pH 6.8 with 1 N NaOH, and was used as the sample for electrophoresis.

### b) Reduction alone

Humanized PTHrP antibody solution and SDS sample buffer for reduction (5% 2-ME, 5% SDS, 2 mM EDTA, 0.005% BPB, 20 mM Tris-HCl, pH 8.0) were mixed at a ratio of 1: 5 (v/v) while stirring, denaturation and reduction were carried out by heating at 95°C in a water bath for 5 minutes, and this was used as the sample for electrophoresis.

### 2) Separation of H-chains and L-chains by SDS-PAGE

H-chains and L-chains were separated electrophoretically by SDS-PAGE. Electrophoresis was performed according to the method of Laemmli. SDS-PAGE mini 12% (TEFCO) was used as the gel. Electrophoresis was performed in running buffer (0.1% SDS, 200 mM Glycnie, 25 mM Tris) at 25 mA (constant current) , and was terminated when BPB had completely phoresed. The bands of each chain were detected upon staining with staining solution (0.2% Coomassie Brilliant Blue R-250, 40% ethanol, 10% acetic acid). After destaining with destaining solution (40% methanol, 10% acetic acid) , the gel was stored in a refrigerator by soaking in Milli Q water.

### 3) In-gel digestion

Portions corresponding to each of the bands of the H-chains and L-chains were excised, digestion buffer (0.1% SDS, 100 mM Tris-HCl, pH 9.0) enough to soak the gel was added, and this was pre-incubated in a thermoregulated air bath at 37°C for 30 minutes. Lys-C enzyme was added to a final concentration of 5 µg/ml, and digestion was carried out in a thermoregulated air bath at 37°C for 18 hours. Lys-C specifically cleaves the C-termini of lysines in the amino acids constituting the protein.

### 4) Extraction of fragments from the gel

The digest solution was poured into another tube, elution buffer (0.1% SDS, 100 mM Tris-HCl, pH 9.0) was freshly added, and was eluted in a thermoregulated air bath at 37°C for 1 hour. This elution process was repeated 3 times, and the obtained digest solution and elute were mixed and then used as a digest mixture solution in the following analysis.

### 5) Confirmation of reduction-carboxymethylation and digestion by mass spectrometry (TOF-MS)

The digest mixture solution was desalted using ZipTip (MILLIPORE) to prepare a TOF-MS sample. 3,5-dimethoxy-4-hydroxycinnamic acid (Sinapinic Acid) was used as the matrix. The sample and matrix were dissolved in a 50% acetonitrile solution containing 0.1% trifluoroacetic acid (TFA), and was dried on a sample plate. Voyager-DE STR (PerSeptive Biosystems) was used as the instrument. This instrument utilizes the matrix-assisted laser desorption ionization (MALDI) method as the ionization method and time-of-flight (TOF) type for detection.

Method file that can cover the range of theoretical mass numbers for the sample was selected for the measurement. Conditions for such measurements were: accelerated voltage: 20 kV; delay: 30 ns; and mode: linear, positive. All operations were performed according to the manual.

### 6) Fractionation of fragments by high-performance liquid chromatography (HPLC)

The digest mixture solution was subjected to RP-HPLC, and the digested fragments were fractionated.

ODS-A (250x 4.6 mm) (YMC) was used as the separation column, and TSKguardgel DEAE-SW (10x 6.0mm) (Tosoh) was used as the guard column. SCL-6A System Controller, LC-6A Pump, SPD-6A UV Detector, and C-R3A Chromatopac integrator (Shimadzu) were used as equipments.

Milli Q water and acetonitrile containing 0.1% TFA were used as the mobile phase. Elution was carried out at a flow rate of 1.0 ml/min using a 5-60% linear gradient of acetonitrile. Detection was carried out at a UV frequency of 215 nm.

After freeze drying, the fractions were dissolved with small amounts of formic acid to prepare samples for fragment identification using an amino acid sequencer and TOF-MS.

### 7) Identification of fragments using an amino acid sequencer

The fractionated samples were placed directly onto sample filters, dried, and then analyzed. Protein Sequencer 476A (Applied Biosystems) was used as the instrument, and all operations were performed according to the manual.

### 8) Identification of fragments using TOF-MS

The fractionated samples were dried on sample plates. The matrix (Sinapinic acid) dissolved in 50% acetonitrile solution containing 0.1% TFA was dropped on each sample to prepare the analysis sample. The same instrument as that used in 5) was used. Method file that can cover the range of theoretical mass numbers for the sample was used for the measurement. Conditions for the measurement were: accelerated voltage: 20 kV; delay: 100 ns; and mode: linear, positive. All operations were performed according to the manual.

### (Results)

### 1) Reduction and carboxymethylation of S-S bonds

Humanized PTHrP antibody is an IgG1 antibody, and forms a structure wherein two each of the H-chains and 1-chains are cross-linked by S-S bonds. When the S-S bonds are reductively cleaved leaving the highly reactive SH groups as they are, reorganization of the S-S bonds or addition of reagents or such will occur which makes interpretation of data complicated and laborious. Therefore, this system is inappropriate for mapping. Thus, to avoid unnecessary confusion, incorporation of a treatment for reductive modification of the S-S bonds to the process was considered. Three objects, the sample before electrophoresis, the gel before enzymatic digestion, and the digest mixture solution after elution, were considered as the candidates that should be subjected to the treatment. According to preliminary studies, it had been confirmed that something is already attached to the SH group in the digest mixture solution after elution from the gel (data not shown). Thus, this object was rejected as the candidate.

Generally, reductive modification is carried out in gel. However, herein, reduction and carboxymethylation were performed on samples before electrophoresis. A sample before electrophoresis was denatured in SDS sample buffer, i.e., in SDS, and then reduction and carboxymethylation treatments were performed. The treated sample could be directly phoresed, and the migration pattern was not affected by the treatment. Even in TOF-MS of digest mixture solutions, fragments involved in S-S bonds were detected in accordance with the theoretical mass numbers, and carboxymethylation reaction had progressed. Problems arose neither in the chromatogram of RP-HPLC nor to the amount of recovery.

### 2) Comparison of the presence and absence of carboxymethylation treatment

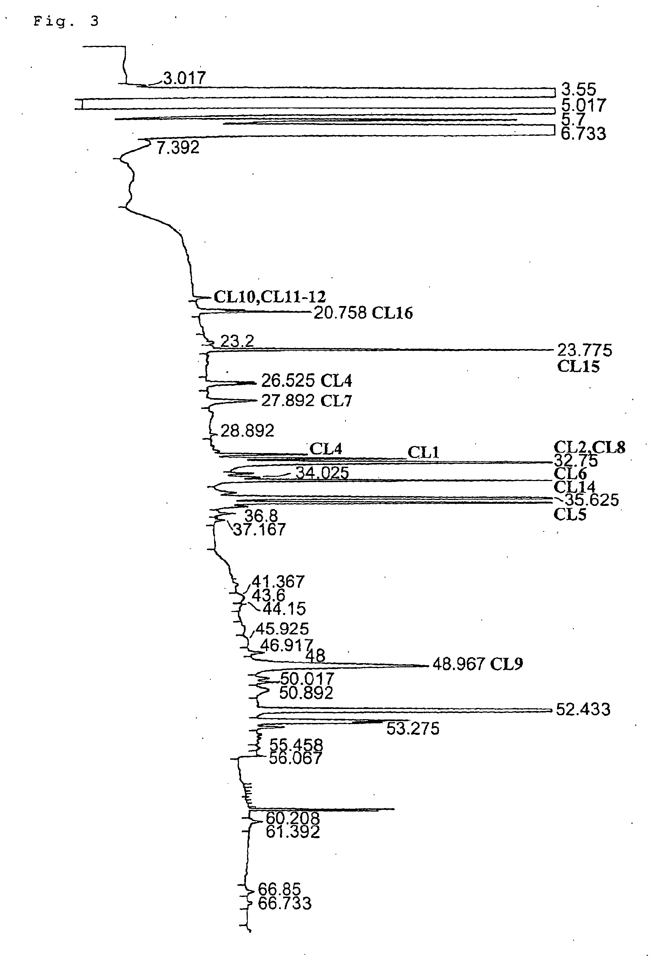

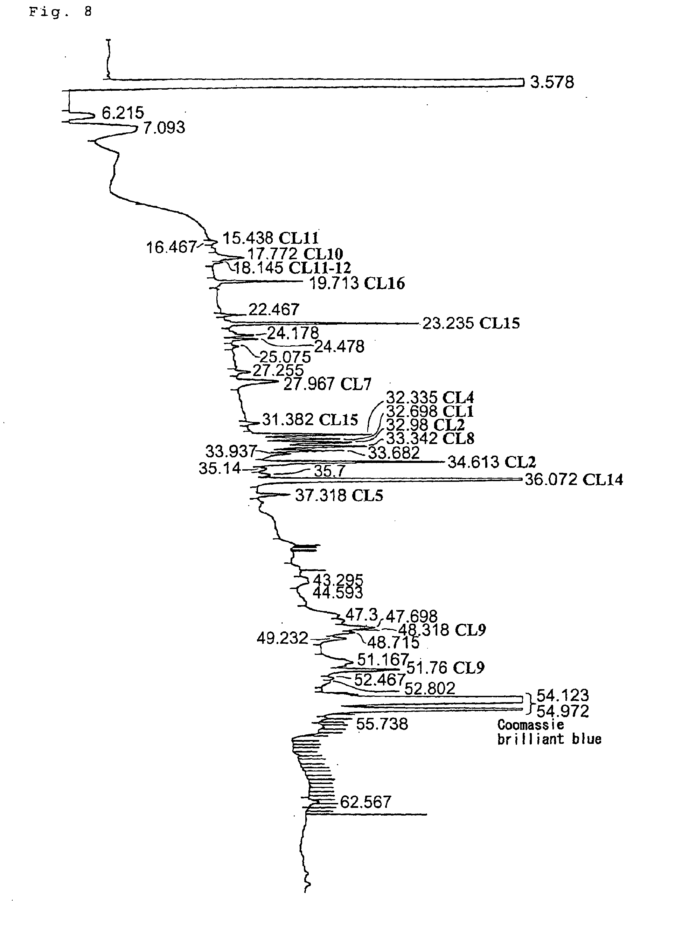

Comparison of chromatograms with and without carboxymethylation treatment is indicated in Fig. 8 (without carboxymethylation treatment) and Fig. 3 (with carboxymethylation treatment). Except the carboxymethylation treatment, all manipulations were conducted following the method of the present invention.

The results obtained by the sequencer and TOF-MS on the fractions suggested that reagents and such are added to the SH group in peak fragments involved in S-S bonds. The same sequence detected in several positions was considered to correspond to the different SH group adducts, according to the results of TOF-MS. Furthermore, all fragments not involved in S-S bonds were detected in the same manner in both Figs. 8 and 3.

Finally, separation of the H-chains and L-chains by SDS-PAGE under reducing conditions, in-gel fragmentation, and structure analysis were found to be possible even without the carboxymethylation treatment. Therefore, when just a partial knowledge of the internal sequence of an antibody molecule is required, the amino acid sequence without the cysteine residues can be determined. Carboxymethylation treatment was performed for the following experiments and results, except otherwise stated.

### 3) TOF-MS analysis of digest mixture solution

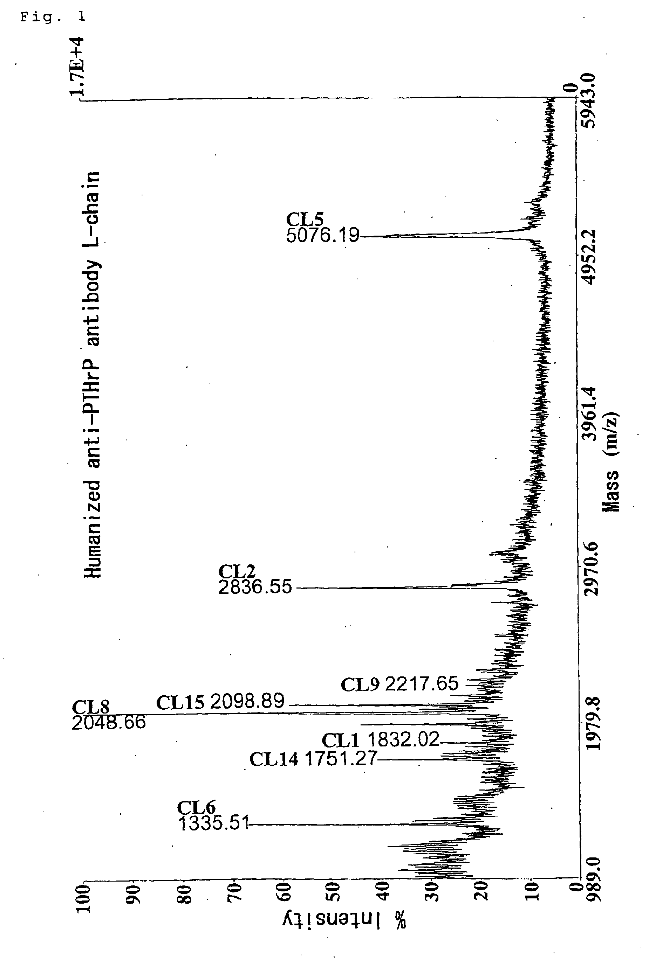

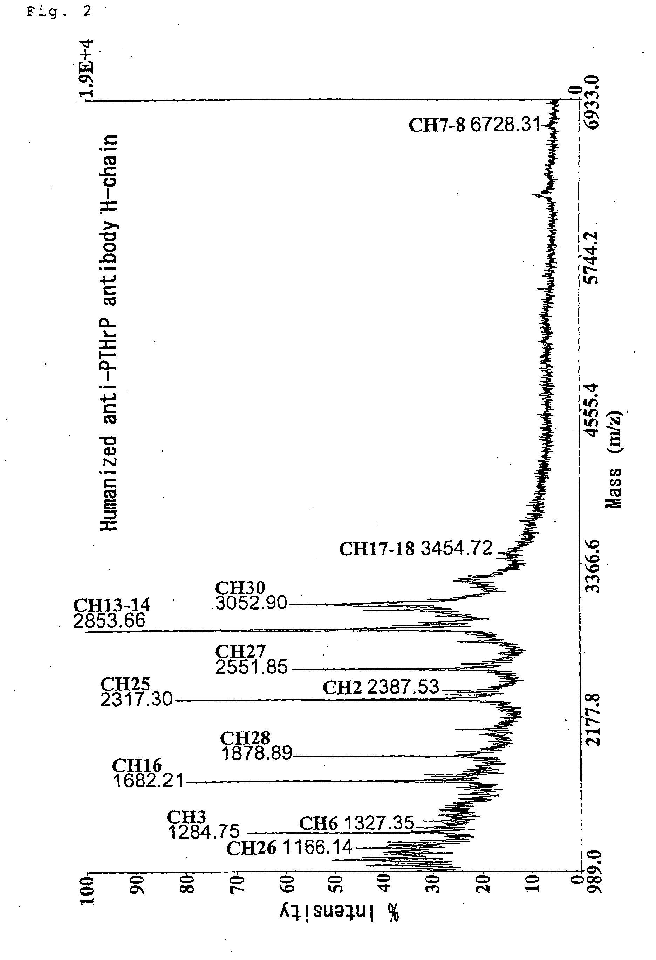

After reduction and carboxymethylation treatments and SDS-PAGE of the humanized PTHrP antibody, the bands for the H-chain and L-chain were excised, and the gel was directly digested with Lys-C enzyme. Sinapinic Acid was used as the matrix, and the examination results by TOF-MS for the digests mixture of each chain are shown in Figs. 1 and 2. The numbers at the peak tops indicate the measured mass and fragment numbers presumed to corresponding thereto.

7 of 16 fragments predicted from the sequence of the L-chain were detected (Fig. 1). Fragments that should be theoretically generated according to the amino acid sequence of the L-chain (SEQ ID NO: 1) are shown in Table 1. The table shows from the left, the fragment number (CLn), amino acid sequence of the fragment, and the theoretical mass. Furthermore, "constant" and "variable" indicate the part that divides the respective constant region and variable region. The seven detected peaks had nearly the theoretical mass, and included a conjectural N-terminal blocked fragment (CL1) that could not be identified with the sequencer.

The peak of CL9 was small and its existence was unclear. Difficulty in the detection of fragments having low ionization efficiency is inevitable in TOF-MS. The existence of CL9 was confirmed due to the analysis of fractions using a sequencer. Therefore, it was determined that the CL9 was hardly detected due to its low ionization efficiency. The other eight fragments that were not detected are fragments with a mass of 1,000 or less. These fragments were regarded as undetected because their peaks were lost in the noise of the low molecular weight region or in the peaks of extracts from the reagents and gel, and consequently their assignments were unclear.

**Table 1**

| | | | |
|---|---|---|---|
| CL3 | 43-45 | GPK | 300.3581 |
| CL12 | 173-175 | PSK | 330.3844 |
| CL13 | 176-180 | QSNNK | 589.6063 |
| CL10 | 159-165 | ADGSP VK | 672.7364 |
| CL11 | 166-172 | AGVET TK | 704.7786 |
| CL4 | 46-51 | YLMDL K | 781.9716 |
| CL7 | 112-119 | LTVLG QPK | 855.0456 |
| | | variable ← → constant | |
| CL16 | 214-221 | TVAPT EBS | 864.9289 |
| CL6 | 100-111 | EQFVY VFGGG TK | 1331.4912 |
| CL14 | 181-195 | YAASS YLSLT PEQWK | 1743.9342 |
| CL1 | 1-19 | XLVLT QSPSA SASLG ASVK | 1827.0656 |
| CL8 | 120-138 | ANPTV TLFPP SSEEL QANK | 2043.2618 |
| CL15 | 196-213 | SHRSY SBQVT HEGST VEK | 2093.2192 |
| CL9 | 139-158 | ATLVB LISDF YPGAV TVAWK | 2212.5954 |
| CL2 | 20-42 | LTBTL SSQHS TYTIE WYQQQ PEK | 2830.0820 |
| CL5 | 52-99 | QDGSH STGDG IPDRF SGSSS GAERY LTISS LQSED EADYI BGVGD TIK | 5068.3031 |

15 out of 31 fragments predicted from the sequence of the H-chain were detected with nearly the theoretical mass (Fig. 2). The fragments predicted from the amino acid sequence of the H-chain (SEQ ID NO: 2) are shown in Tables 2 and 3. The tables show from the left: the fragment number (CHn) , amino acid sequence of the fragment, and the theoretical mass. Furthermore, "constant" and "variable" indicate the part that divides the respective constant region and variable region. Among the observed peaks, CH7-8, 13-14, 17-18 were detected as linked fragments wherein the Lys-Pro sequences that are believed to be difficult to cleave with Lys-C had not been cleaved.

Except fragments with a mass of 1,000 or less whose assignments are unclear due to the same reasons as for the above-mentioned L-chain, fragments that could not be detected were CH4 and CH15. However, the existence of both of these fragments was confirmed by analyzing these fractions with a sequencer. Thus, it was suggested that these fragments could not be detected due to their low ionization efficiency.

Most types of fragments could be recovered from the gel by the current method. Therefore, enzymatic digestion in the gel was considered to be possible. Furthermore, in terms of amount, 80% to 90% of the fragments were estimated to be recovered according to the spectrum intensity of TOF-MS.

**Table 2**

| | | | |
|---|---|---|---|
| CH10 | 215-215 | K | 146.1894 |
| CH24 | 340-341 | AK | 217.2683 |
| CH14 | 248-249 | PK | 243.3062 |
| CH17 | 290-291 | TK | 247.2946 |
| CH 20 | 322-323 | BK | 307.3709 |
| CH 9 | 212-214 | VDK | 360.4107 |
| CH 19 | 319-321 | EYK | 438.4810 |
| CH 21 | 324-327 | VSNK | 446.5042 |
| CH23 | 336-339 | TISK | 447.5323 |
| CH 11 | 216-219 | VEPK | 471.5543 |
| CH 12 | 220-223 | SBDK | 509.5378 |
| CH 8 | 207-211 | PSNTK | 545.5934 |
| CH 29 | 411-415 | LTVDK | 574.6753 |
| CH 31 | 441-447 | SLSLS PG | 659.7376 |
| CH 22 | 328-335 | ALPAP IEK | 838.0150 |
| CH 26 | 362-371 | NQVSL TBLVK | 1162.3732 |
| CH 5 | 123-134 | GPSVF FLAPS SK | 1186.3738 |
| CH 3 | 66-76 | GRFTI SRDNS K | 1280.4069 |
| CH 6 | 135-148 | STSGG TAALG BLVK | 1322.5028 |
| CH16 | 276-289 | FNWYV DGVEV HNAK | 1677.8371 |
| CH 28 | 394-410 | TTPPV LDSDG SFFLY SK | 1874.0778 |
| CH 25 | 342-361 | GQPRE PQVYT LPPSR DELTK | 2311.5809 |
| CH 2 | 44-65 | GLEWV ATISS GGSYT YYPDS VK | 2380.5937 |
| CH 27 | 372-393 | GFYPS DIAVE WESNG QPENN YK | 2544.6738 |
| CH 13 | 224-247 | THTBP PBPAP ELLGG PSVFL FPPK | 2622.0617 |
| CH 15 | 250-275 | DTLMI SRTPE VTBVV VDVSH EDPEV K | 2957.3307 |

**Table 3**

| | | | |
|---|---|---|---|
| CH30 | 416-440 | SRWQQ GNVFS BSVMH EALHN HYTQK . | 3046.3577 |
| CH18 | 292-318 | PREEQ YNSTY RVVSV LTVLH QDWLN GK | 3232.6046 |
| CH1 | 1-43 | XVQLV ESGGG WQPG RSLRL SBAAS GFTFS SYGMS WVRQA PGK | 4528.1109 |
| CH4 | 77-122 | NTLYL QMNSL RAEDT AVYYB ARQTT MTYFA YWGQG TLVTV SS AST K | 5264.9055 |
| | | variable← → constant | |
| CH7 | 149-206 | DYFPE PVTVS WNSGA LTSGV HTFPA VLQSS GLYSL SSVVT VPSSS LGTQT YIBNV NHK | 6189.8668 |

In the above study, TOF-MS measurements of the digest mixture were used to confirm the extent of progress of the reduction and carboxymethylation reactions. As a result, within the measurable range, most of the peaks corresponding to the fragments were detected in accordance with the theoretical mass. Therefore, this spectrum may be utilized for mass mapping. When the sequence is known, the theoretical mass of the fragment can be calculated, and thus the measured value can be assigned. The type of modification can be predicted from the shift of mass even when the modification is one that cannot be detected with a sequencer such as that of a N-terminus modified fragment, or when the modification does not change the RP-HPLC elution position. A more reliable mapping data can be obtained using both TOF-MS spectra and RP-HPLC chromatograms.

### 4) RP-HPLC chromatogram and analysis of the fractions

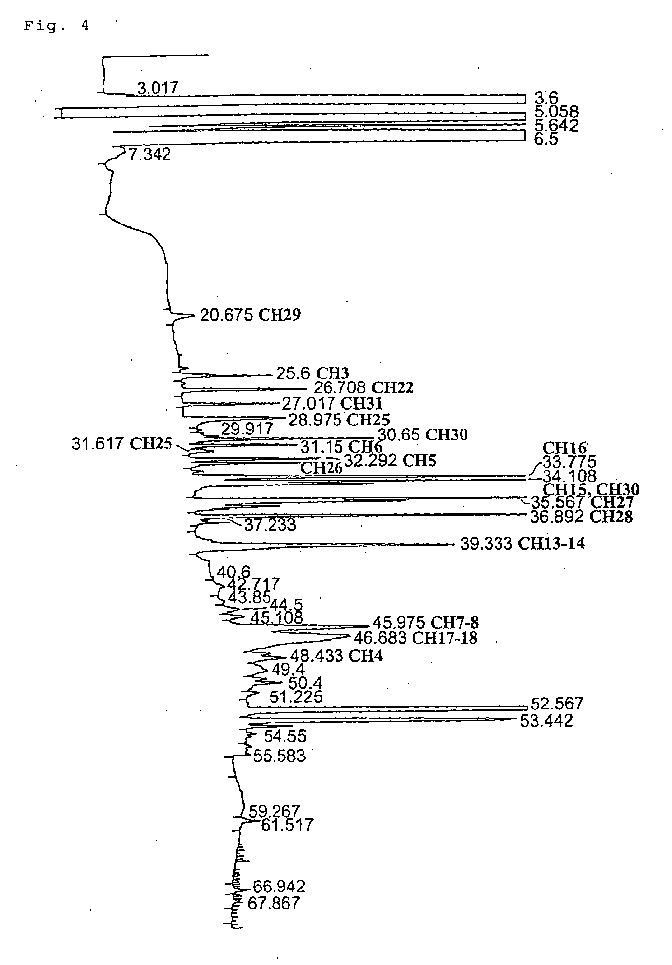

Chromatograms of the digest mixture solutions fractionated by RP-HPLC are shown in Figs. 3 and 4. The fraction number assigned by examining the amino acid sequence of the fraction using a sequencer is indicated at the peak tops. Since there is a limit to the number of residues that can be analyzed using a sequencer, whether or not a long fragment for which the sequence could not be read to the end has the full length of the predicted sequence was determined by combining the result of examination of the fraction by TOF-MS.

13 out of 16 fragments of the predicted sequence could be assigned for the L-chain (Fig. 3). CL11-12 was identified in the form of a linked fragment as the 19.5-minute peak. This was because the Lys-Pro sequence was not cleaved by Lys-C. Furthermore, CL10 was detected simultaneously in this fraction. Similarly, CL2 and CL8 were detected simultaneously at the 32.8-minute peak.

Furthermore, the sequence of CL4 was read from both the 26.5-minute and 32.2-minute peaks. The recovery of the residue Met48 of the 26.5-minute peak was extremely poor in the sequence analysis results, which suggested the occurrence of modification at the Met. From the result of LC-MS, the low recovery was suggested to be the result of the generation of oxidized form of Met. CL9 was identified as the 48.9-minute peak (data not shown), and revealed that the inability of detection by TOF-MS of the digest mixture solution was due to problems with ionization efficiency. Among the fragments that could not be assigned, CL1 could not be identified using a sequencer due to pyroglutamylation of the N-terminal Gln. However, the 32.5-minute peak was regarded as CL1 from the fact that nothing could be read from this peak using a sequencer and the results of TOF-MS of this fraction. CL3 and CL13 that could not be assigned are small fragments comprising 3 and 5 amino acid residues, respectively. Thus, they were supposed to have been passed through and were not retained by RP-HPLC using the ODS column.

19 out of 31 fragments from the predicted sequence could be assigned for the H-chain (Fig. 4). CH13-14 and CH17-18 were identified as the 39.3-minute and 46.7-minute peaks, respectively. This was because the Lys-Pro sequence was not cleaved by Lys-C. This does not oppose the fact that peaks with masses corresponding to the linked fragments were detected in the TOF-MS of the digest mixture solution (Fig. 2). Although the sequence of CH7 was read from the 45.9-minute peak, this fragment was too long to confirm the full length sequence. However, CH7 was regarded as a fragment linked to CH8 due to the facts that CH7 has a Lys-Pro sequence, a sequence difficult to cleave with Lys-C, and a peak with a mass for a linked fragment was detected by TOF-MS.

CH15 and CH30 were detected simultaneouly in the 34.1-minute peak. The sequence of CH30 was read from both 30.6-minute and 34.1-minute peaks, and the amount of recovery of the residue Met429 from the 30.6-minute peak was particularly poor in the sequence analysis results. Thus, the Met seemed to have been modified. Similarly to CL4, this was suggested to be caused by the formation of the oxidized form of Met. CH4 and CH15 were identified as the 48.4-minute and 34.1-minute peaks, respectively, revealing that the inability of detection by TOF-MS of the digest mixture was due to problems with ionization efficiency.

A good reproducibility was confirmed for the above-mentioned RP-HPLC chromatogram. Furthermore, recovery of most of the fragments was confirmed from mass spectrometry by TOF-MS and sequence analysis using a sequencer. Furthermore, the recovery of the fragments calculated using the amount of applied sample that were calculated from the yields of repeated analysis using the sequencer was good with a recovery of 30% to 60%. From the above, the above system nearly fulfils the essential conditions for producing a mapping system. Therefore, the present method that uses the in-gel digestion method can be utilized as a peptide mapping system for antibodies.

### 5) Examples of detection of heterogeneous fragments (glycosylation)

Glycosylation of the residue Asn298 of the H-chain is predicted in humanized PTHrP antibody. The residue 298 exists in the CH17-18 fragment, whose peak was at 46.6 minutes (Fig. 4) according to the analysis by a sequencer.

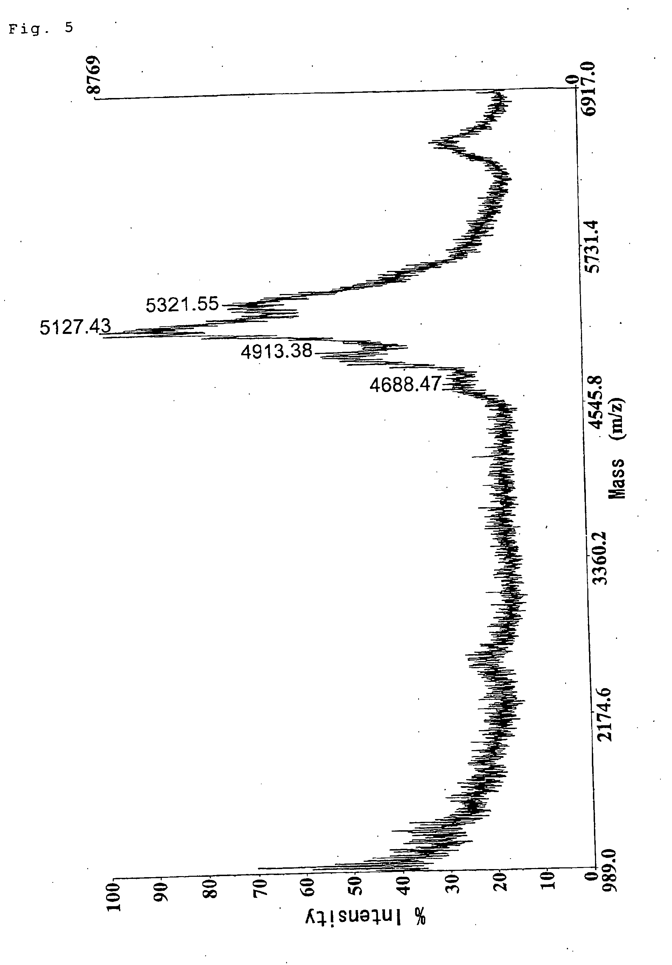

The broad shape thereof compared to other fragment peaks is suggested to be the result of heterogeneity of sugar chains. The result of TOF-MS of this fraction is shown in Fig. 5. No peaks were detected near the theoretical mass of 3461 corresponding to the peptide fragment portion alone, and peaks were detected near 4500 to 6900. The shift in mass corresponds to the added sugar chains, and the observed multiple peaks suggests that the heterogeneity of the sugar chain was detected. Furthermore, using this fraction, information relating to sugar chains can be obtained by combining mass spectrometry and glycosidase digestion.

### 6) Determination of the subclass of H-chain and the type of L-chain

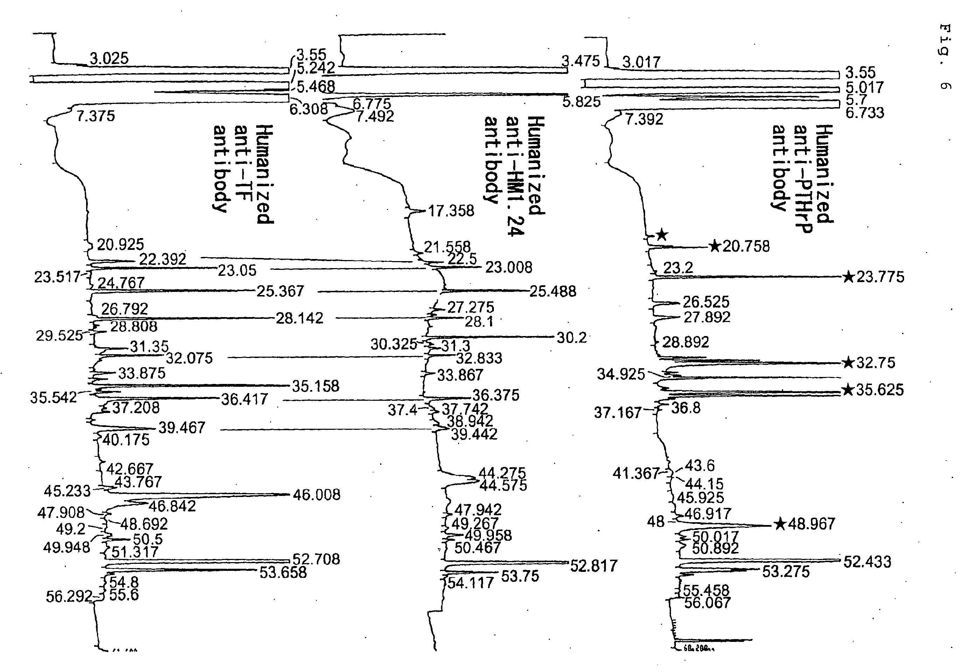

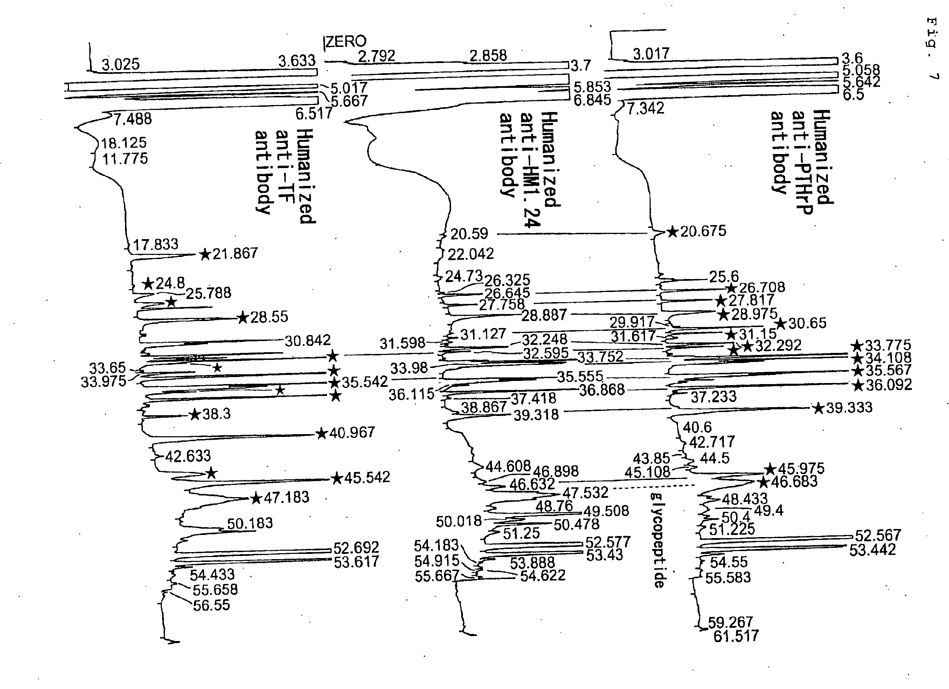

Humanized antibodies except humanized PTHrP antibody, i.e., humanized anti-HM1.24 antibody and humanized anti-TF antibody, were analyzed according to the method of this invention to obtain their chromatograms. Humanized anti-HM1.24 antibody and humanized anti-TF antibody were produced according to WO98/1458 and WO99/51743, respectively. Comparison of the chromatograms of the L-chains and H-chains of each antibody are shown in Fig. 6 and Fig. 7, respectively. Peaks at the same detection positions with similar appearance on the chromatograms were connected with straight lines.

In Fig. 6, several peaks of humanized anti-HM1.24 antibody and humanized anti-TF antibody could be connected with straight lines but none of the humanized PTHrP antibody. According to the predicted nucleotide sequence of cDNA, the type of L-chain is known to be the same, κ-chain, for humanized anti-HM1.24 antibody and humanized anti-TF antibody, and different, λ-chain, for humanized PTHrP antibody. Furthermore, according to the analysis results of fractions from humanized anti-TF antibody, all peaks that could be connected with straight lines were confirmed to correspond to fragments derived from the constant region. In the figure, peaks marked with stars ( 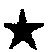 ) correspond to fragments of the constant region. These findings revealed that, when antibodies of the same type are analyzed, fragments derived from the constant region are detected in a similar manner, thus showing similar chromatograms; the chromatogram of a different type of antibody will be different.

In Fig. 7, many peaks for the humanized PTHrP antibody and humanized anti-HM1.24 antibody could be connected with straight lines, but only two lines could be drawn to the peaks for the humanized anti-TF antibody. According to the predicted nucleotide sequence of cDNA, the subclass of H-chain is γ1 for humanized PTHrP antibody and humanized anti-HM1.24 antibody, and γ4 for humanized anti-TF antibody. Furthermore, according to the analysis results of fractions from humanized PTHrP antibody, all peaks that could be connected with straight lines were confirmed to correspond to fragments derived from the constant region. Analyses of humanized anti-TF antibody fractions have confirmed that peaks that could be connected to those of the humanized anti-TF antibody correspond to fragments derived from a segment that has the same sequence in the constant regions, γ1 and 4. In a similar manner to the analysis of the type of L-chains, this revealed that similar chromatograms are obtained by the same subclass and different chromatograms by different subclasses.

Conversely, comparison of chromatograms allows prediction of the subclass and type of an antibody. It has been known that unidentified antibodies, and even a mixture of antibodies, can be identified by comparing chromatograms.

When the subclass and type of an antibody are identified, the peaks derived from the variable region can be specified. Thus, efficient structural analysis of an antibody can be conducted by preferentially analyzing those peaks.

### 7) Required amount of sample

50 µg of samples were used in this examination. However, for the confirmation of a sequence, 20 µg or so is sufficient, and comparison of peptide maps is possible even with 10 µg. With the use of microbore HPLC and such, analysis may be conducted with even smaller quantities of samples. Compared to conventional methods, which require several hundred micrograms of samples, analysis can be performed using an extremely small amount of sample. The present method technically enables preparation of samples with an even smaller amount, and the minimum amount necessary at the start of the experiment depends on the sensitivity of analyzer (sequencer, mass spectrometer, etc.) used for identification. By using a highly sensitive analyzer, much smaller quantities of samples can be analyzed and identified.

The present method enables simultaneous and easy separation and purification of H-chains and L-chains that was difficult according to conventional methods. Enzymatic digestion in gel, fragment recovery from the gel, and sequence analysis of the recovered fragments were shown to be possible under the present conditions. Since most of the sequence can be identified from the recovered fragments, the method was found out to be useable to identify the amino acid sequence of antibodies. Furthermore, the recovery rate, which was generally believed to be the problem in the in-gel digestion until now, was good with a value of 30% to 60% or so. The reproducibility of the results was also good, and the present method that utilizes the in-gel digestion can be used as a peptide mapping system for antibodies.

### [Example 2]

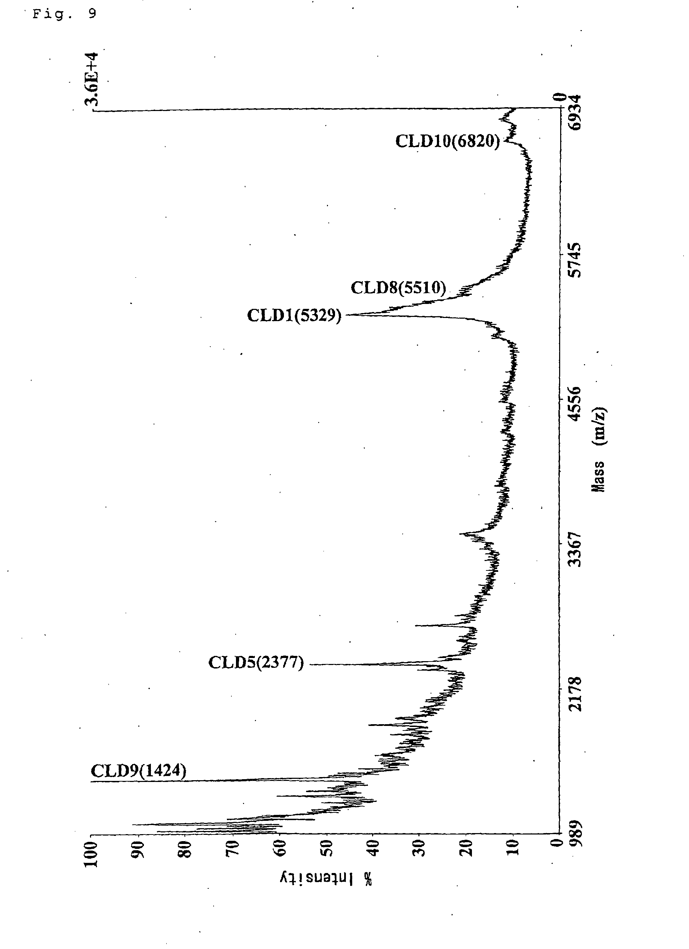

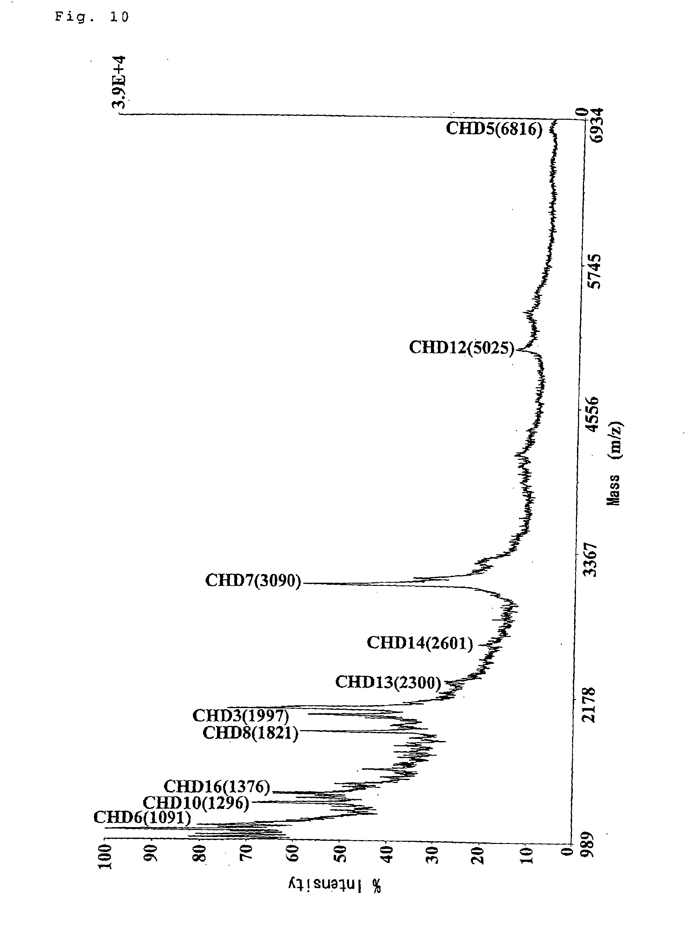

With the exception that (1) endoproteinase Asp-N was used as the digestive enzyme, and (2) 50 mM sodium phosphate buffer (pH 8.0) was used as the digestion buffer, the following three types of antibodies were analyzed under the same conditions as described in Example 1. Humanized anti-IL6-R antibody was produced according to WO92/19759. Asp-N is an endoproteinase that cleaves the N-terminal side of aspartic acid.
Humanized anti-PTHrP antibody
Humanized anti-TF antibody
Humanized anti-IL6-R antibody
The results of analysis on the digest mixture of the H-chains and L-chains of humanized anti-PTHrP antibody are shown in Fig. 9 (L-chain) and Fig. 10 (H-chain). The fragment numbers and theoretical mass (in parenthesis) are indicated at the peak tops for the assigned peaks. For the L-chain, 5 out of 10 fragments that were predicted from the amino acid sequence had been detected. On the other hand, for the H-chain, 10 out of 17 fragments that were predicted from the amino acid sequence had been detected. The fragments predicted from the amino acid sequence of the L-chain (SEQ ID NO: 1) are shown in Table 4, and the fragments predicted from the amino acid sequence of the H-chain (SEQ ID NO: 2) are shown in Table 5. The tables indicate from the left, fragment number (CLn), amino acid sequence of the fragment, and the theoretical mass. For both of the L-chain and H-chain, the mass of the detected fragments were nearly in accordance with the theoretical mass.

The other five undetected fragments of the L-chain had a mass of 1000 or less, and were regarded as undetected because their peaks were lost in the noise of the low molecular weight region or in the peaks corresponding to the extracts from the reagents and gel, and consequently their assignments were unclear. In the H-chain, for the same reason, fragments having a mass of 1000 or less were regarded as undetected.

**Table 4**

| | | | |
|---|---|---|---|
| CLD6 | 86-88 | DEA | 333.2983 |
| CLD4 | 60-63 | DGIP | 400.4321 |
| CLD2 | 49-52 | DLKQ | 502.5684 |
| CLD3 | 53-59 | DGSHSTG | 659.6106 |
| CLD7 | 89-95 | DYIBGVG | 783.8574 |
| CLD9 | 147-159 | DFYPGAVTVAWKA | 1424.6195 |
| CLD5 | 64-85 | DRFSGSSSGAERYLTISSLQSE | 2377.5076 |
| CLD1 | 1-48 | XLVLTQSPSASASLGASVKLTBTLS SQHSTYTIEWYQQQPEKGPKYLM | 5329.0092 |
| CLD8 | 96-146 | DTIKEQFVYVFGGGTKLTVLGQPKA | 5510.3502 |
| | | variable←→constant | |
| | | NPTVTLFPPSSEELQANKATLVBLIS | |
| CLD10 | 160-221 | DGSPVKAGVETTKPSKQSNNKYAAS | 6820.4176 |
| | | SYLSLTPEQWKSHRSYSBQVTHEGS TVEKTVAPTEBS | |

**Table 5**

| | | | |
|---|---|---|---|
| CHD15 | 400-401 | DS | 220.1822 |
| CHD9 | 266-270 | DVSHE | 585.5715 |
| CHD6 | 213-221 | DKKVEPKSB | 1091.2510 |
| CHD2 | 62-72 | DSVKGRFTISR | 1265.4356 |
| CHD10 | 271-280 | DPEVKFNWYV | 1296.4453 |
| CHD16 | 402-413 | DGSFFLYSKLTV | 1376.5724 |
| CHD8 | 250-265 | DTLMISRTPEVTBVVV | 1821.1469 |
| CHD3 | 73-89 | DNSKNTLYLQMNSLRAE | 1997.2173 |
| CHD13 | 357-376 | DELTKNQVSLTBLVKGFYPS | 2300.6156 |
| CHD14 | 377-399 | DIAVEWESNGQPENNYKTTPPVL | 2601.8101 |
| CHD7 | 222-249 | DKTHTBPPBPAPELLGGPSVFLFPP KPK | 3090.6154 |
| CHD11 | 281-312 | DGVEVHNAKTKPREEQYNSTYRVVS VLTVLHQ | 3698.1118 |
| CHD17 | 414-447 | DKSRWQQGNVFSBSVMHEALHNHYT QKSLSLSPG | 3931.3429 |
| CHD12 | 313-356 | DWLNGKEYKBKVSNKALPAPIEKTI SKAKGQPREPQVYTLPPSR | 5025.8094 |
| CHD4 | 90-148 | DTAVYYBARQTTMTYFAYWGQGTL VTVSSASTKGPSVFPLAPSSKSTSG | 6202.9944 |
| | | variable←→ constant | |
| | | GTAALGBLVK | |
| CHD1 | 1-61 | XVQLVESGGGWQPGRSLRLSBAAS GFTFSSYGMSWVRQAPGKGLEWVAT | 6461.2157 |
| | | ISSGGSYTYYP | |
| CHD5 | 149-212 | DYFPEPVTVSWNSGALTSGVHTFPA VLQSSGLYSLSSWTVPSSSLGTQT | 6816.5775 |
| | | YIBNVNHKPSNTKV | |

Next, analyses of the digest mixtures were performed by RP-HPLC for humanized anti-PTHrP antibody and humanized anti-IL6-R antibody.

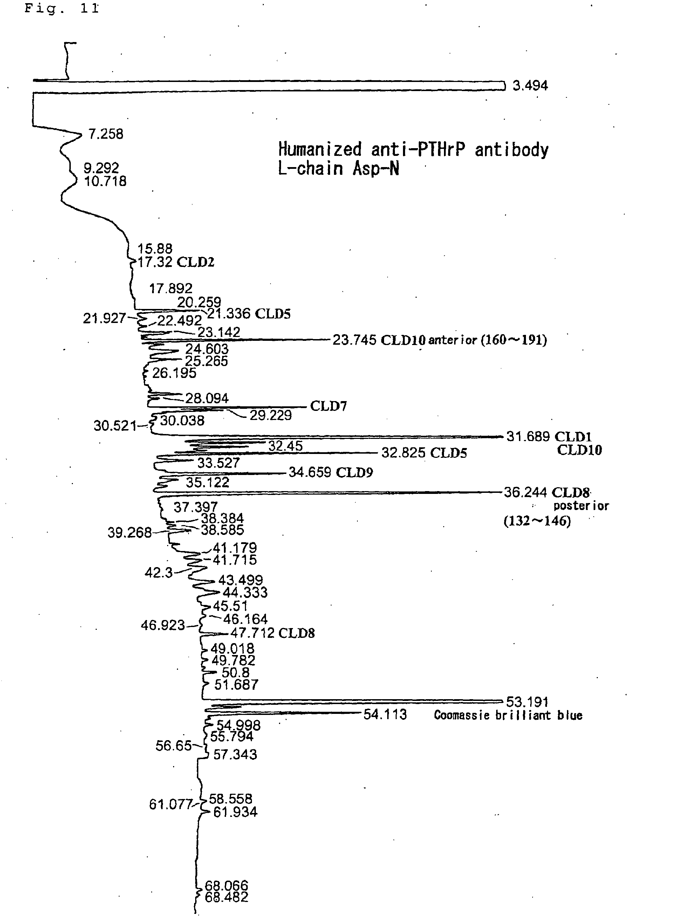

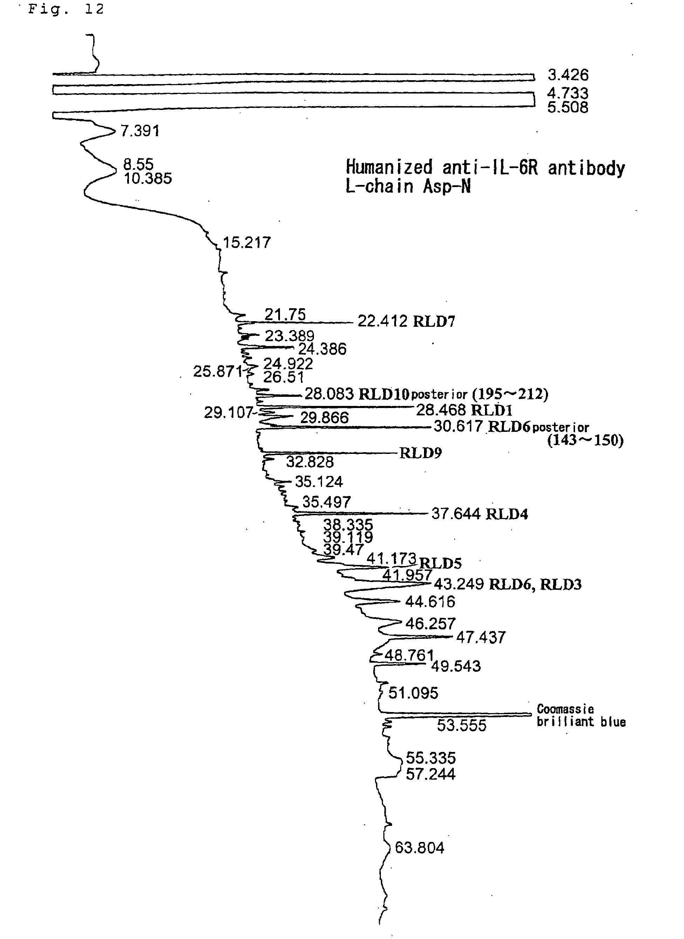

The chromatograms obtained by subjecting the digest mixture solutions to RP-HPLC for fractionation are shown in Figs. 11 and 12. Fig. 11 is the RP-HPLC chromatogram of the Asp-N digest mixture of the L-chain of humanized anti-PTHrP antibody. Fig. 12 is the RP-HPLC chromatogram of the Asp-N digest mixture of the L-chain of humanized anti-IL6-R antibody.

The assigned fragment numbers determined by examining the amino acid sequences of the fractions with a sequencer is shown at the peak tops. The number of residues that can be analyzed with a sequencer is limited. Therefore, whether a long fragment that could not be read through the end has the full length of the predicted sequence was determined in combination with the results of examination on the fraction by TOF-MS.

For the L-chain of humanized anti-PTHrP antibody (Fig. 11), 7 out of 10 fragments from the predicted sequence were assigned. For the L-chain of humanized anti-IL6-R antibody (Fig. 12), 8 out of 10 fragments from the predicted sequence were assigned. Fragments cleaved at the N-terminus of Glu(E) besides Asp(D) were detected as side-reaction products. The substrate specificity of Asp-N used for digestion is incomplete. Thus, formation of such side-reaction products seemed to be inevitable. Fragments predicted from the amino acid sequence of the L-chain of humanized anti-IL6-R antibody are shown in Table 6. The table indicates from the left, fragment number, amino acid sequence of the fragment, and the theoretical mass.

**Table 6**

| | | | |
|---|---|---|---|
| CLD6 | 86-88 | DEA | 333.2983 |
| CLD4 | 60-63 | DGIP | 400.4321 |
| CLD2 | 49-52 | DLKQ | 502.5684 |
| CLD3 | 53-59 | DGSHSTG | 659.6106 |
| CLD7 | 89-95 | DYIBGVG | 783.8574 |
| CLD9 | 147-159 | DFYPGAVTVAWKA | 1424.6195 |
| CLD5 | 64-85 | DRFSGSSSGAERYLTISSLQSE | 2377.5076 |
| CLD1 | 1-48 | XLVLTQSPSASASLGASVKLTBTLS SQHSTYTIEWYQQQPEKGPKYLM | 5329.0092 |
| CLD8 | 96-146 | DTIKEQFVYVFGGGTKLTVLGQPKA | 5510.3502 |
| | | variable ←→ constant | |
| | | NPTVTLFPPSSEELQANKATLVBLIS | |
| CLD10 | 160-221 | DGSPVKAGVETTKPSKQSNNKYAAS SYLSLTPEQWKSHRSYSBQVTHEGS TVEKTVAPTEBS | 6820.4176 |

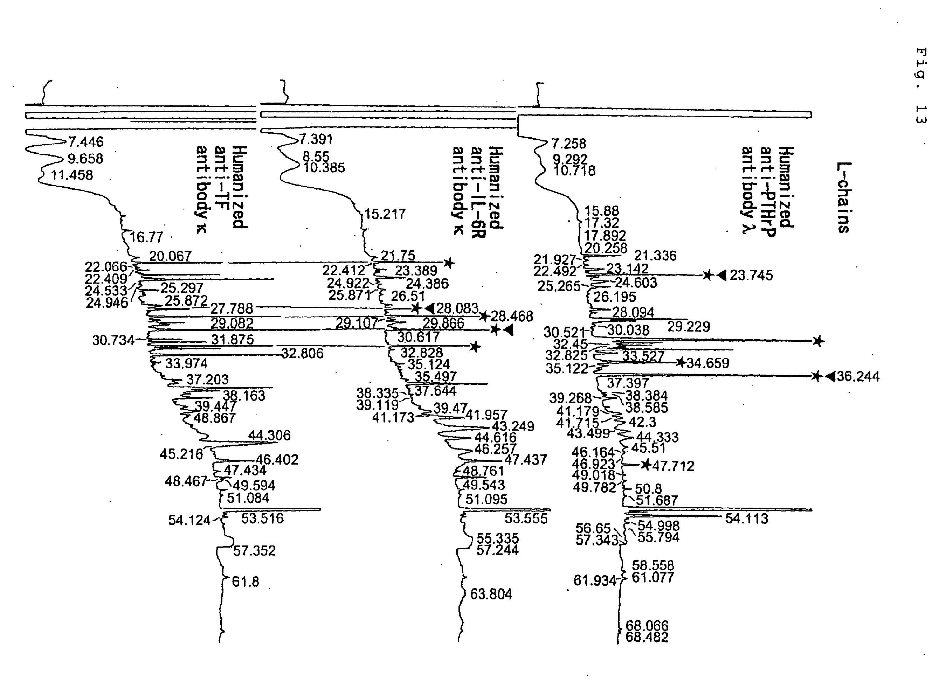

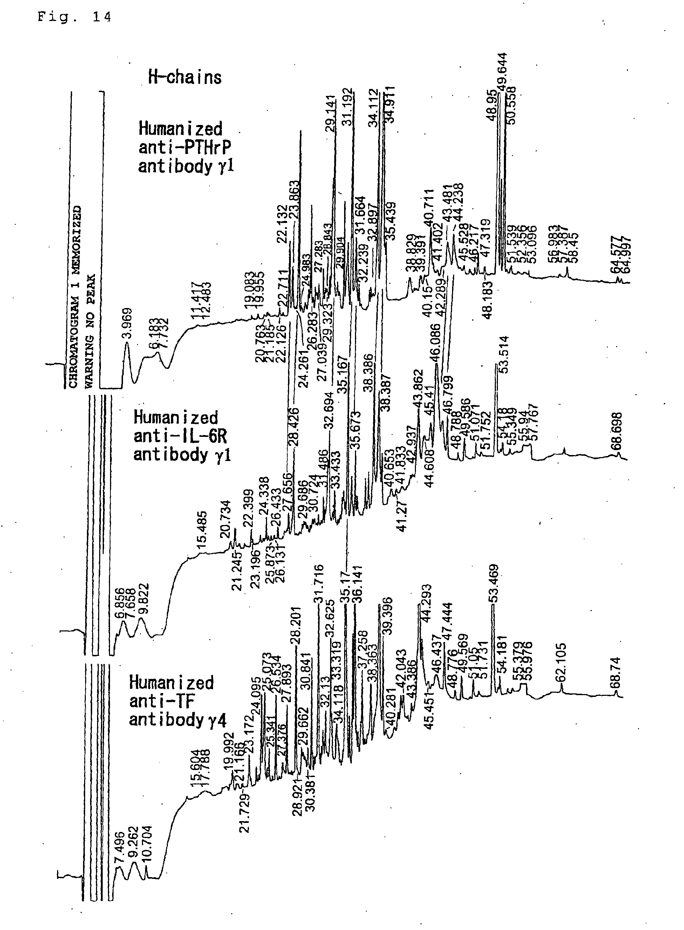

Furthermore, the results of comparison of RP-HPLC chromatograms among the respective H-chains and L-chains of three samples, i.e., humanized anti-PTHrP antibody, humanized anti-TF antibody, and humanized anti-IL6-R antibody, are shown in Fig. 13 (L-chains) and Fig. 14 (H-chains). Peaks at the same detection positions with similar detection patterns were connected with straight lines. In Fig. 12 (L-chains), fragments that could be identified as being derived from the constant region were indicated with stars ( 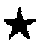 ). Among them, side-reaction products were marked with black upside-down triangles (▼). The results revealed that the same side-reaction products are similarly detected for antibodies of the same type. This shows that information on side-reaction products is also useful for identifying the type of antibodies.

Regarding the H-chain, many straight lines could be drawn between the peaks of humanized anti-PTHrP antibody (Fig. 14, top) and humanized anti-IL6-R antibody (Fig. 14, middle), which antibodies 'belong to the same subclass (γ1). However, only one line could be drawn from the peaks of these antibodies to that of the humanized anti-TF antibody (Fig. 14, bottom). Analysis of fractions using a sequencer confirmed that this peak corresponds to a fragment derived from the portion having a sequence that is the same in both constant regions, γ1 and γ4. Similarly to the case with Lys-C, when digested with Asp-N, the subclass and type of antibody can be predicted by the comparison of chromatograms.

### [Example 3]

Using the L-chain of humanized anti-PTHrP antibody, the amount of sample necessary for the method for analyzing an antibody molecule of the present invention was examined. Under the same conditions as in Example 1, a mixture obtained by digesting the L-chain of humanized anti-PTHrP antibody with Lys-C was analyzed by RP-HPLC.

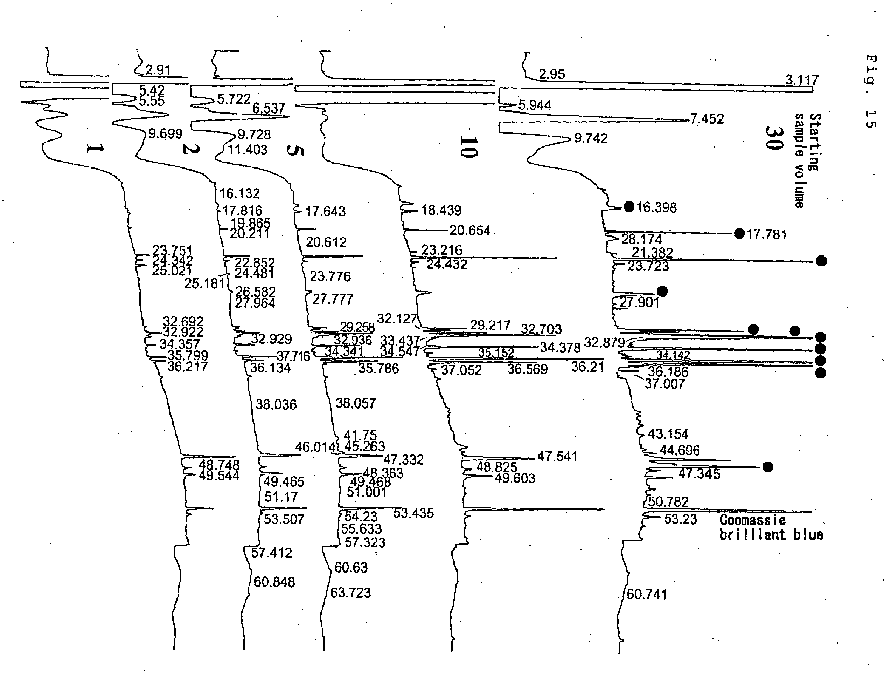

The comparison of RP-HPLC chromatograms, wherein the amounts of L-chain at the start of the experiments were 30, 10, 5, 2, and 1 µg, is shown in Fig. 15. Although the sizes of the peaks changed depending on the amount of sample, the overall shape of the chromatogram did not greatly change. Since the peaks at around 24 minutes, 48 minutes, and 49 minutes had approximately the same size regardless of the amount of sample, these seemed to be contaminants during the process of the analysis procedure. These peaks were not confirmed from the sequencer, and thus were regarded to be other than proteins.

The above-mentioned results confirmed that each fragment can be recovered nearly quantitatively with an amount of sample within the range that were tested in this experiment. Therefore, according to the present invention, even if the amount of sample varies, identification of the subclass and type based on comparison of the chromatograms was considered to be possible. Furthermore, even with an amount of sample of 1 µg, those samples that could be detected as peaks could be fractionated. Furthermore, the amino acid sequence of the fractionated samples could be determined with a sequencer. Therefore, the method for analyzing an antibody molecule based on the present invention was shown to be an excellent analysis method that can be carried out with a small amount of sample of even 1 µg.

Furthermore, the same analysis was performed 5 times on different days and the daily variation of the analysis result was examined. As a result, a high reproducibility was confirmed for the method of this invention.

### [Example 4]

Under the same conditions as in Example 1, the following three types of antibodies were digested with Lys-C, and utilizing TOF-MS and RP-HPLC, the analysis method based on this invention was performed. The results are shown in the following figures. In the figures, the peaks were marked with fragment numbers at the peak tops.

| | TOF-MS/ H-chain | TOF-MS/ L-chain | RP-HPLC/ H-chain | RP-HPLC/ L-chain |
|---|---|---|---|---|
| Humanized anti-HM1.24 antibody | Fig. 16 | Fig. 17 | Fig. 24 | Fig. 25 |
| Humanized anti-TF antibody | Fig. 18 | Fig. 19 | Fig. 26 | Fig. 27 |
| Humanized anti-IL-6R antibody | Fig. 20 | Fig. 21 | Fig. 28 | Fig. 29 |

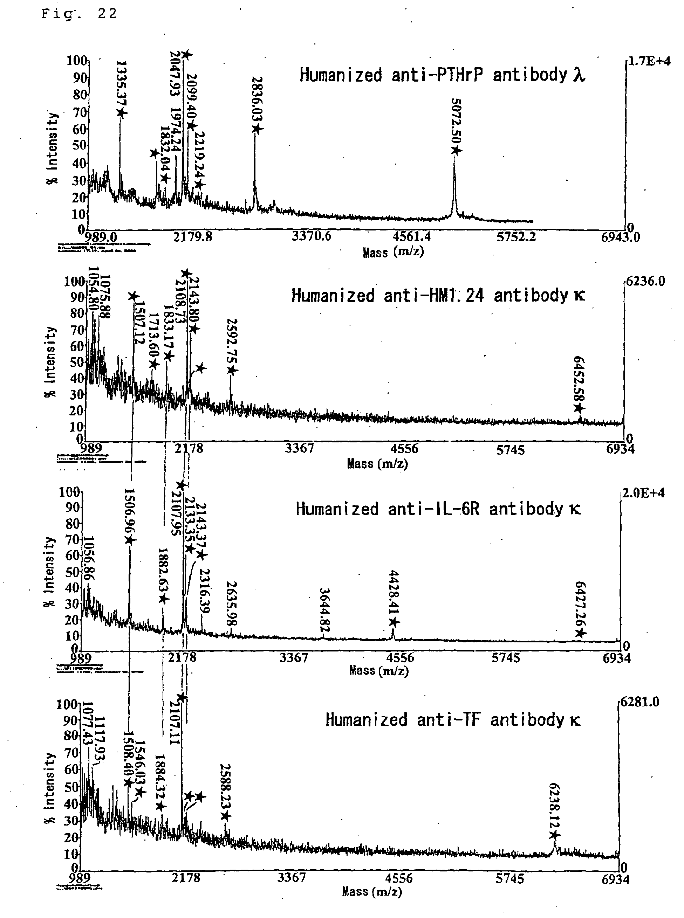

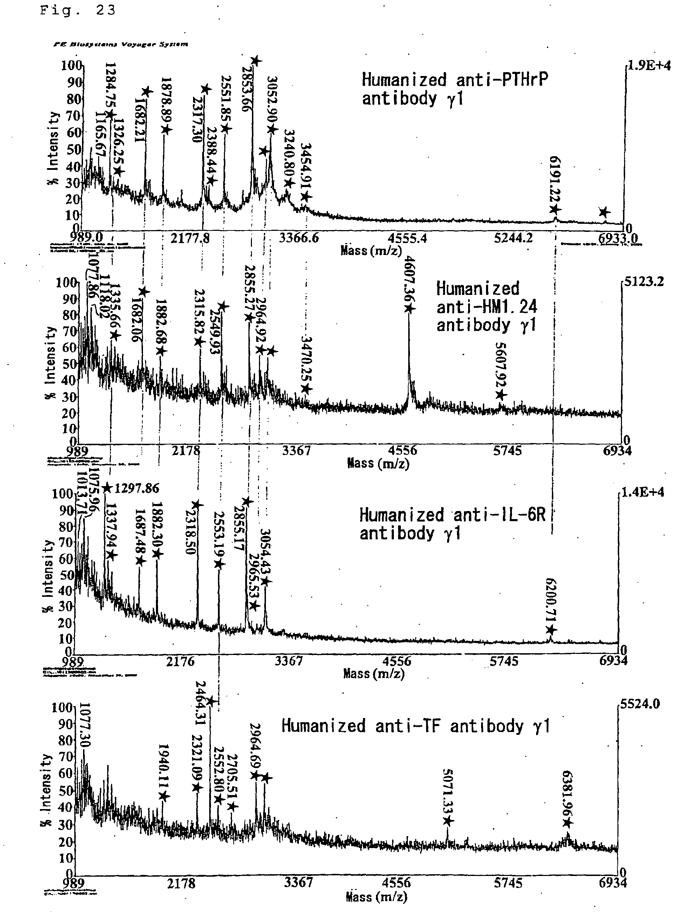

Furthermore, the results of comparison of the chromatograms of TOF-MS for the respective H-chains and L-chains of 4 samples, humanized anti-PTHrP antibody obtained previously in Example 1, and the above-mentioned humanized anti-HM1.24 antibody, humanized anti-TF antibody, and humanized anti-IL6-R antibody are shown in Fig. 22 (L-chains) and Fig. 23 (H-chains). Peaks at the same detection positions with similar detection patterns were connected with a straight line. The peaks were marked with stars ( 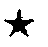 ) at their peak tops.

By TOF-MS of the digest mixture, fragments having a mass of about 1000 or less were undetected because their peaks were lost in the noise of the low molecular weight region or in the peaks corresponding to the extracts of reagents and gel, and consequently assignments were unclear. However, most fragments having a mass of about 1000 or more had been detected. The existence of those peaks that were not detected could be confirmed by analyzing the fractions with a sequencer. Therefore, these fragments were regarded as being difficult to be detected due to their low ionization efficiency. When the spectra of each chain were aligned and compared, the same kinds of antibodies showed similar spectra (Figs. 22 and 23). Therefore, the subclass or type of an antibody can be also identified from TOF-MS spectra. Specifically, TOF-MS measurements of the digest mixture can be utilized for typing of antibodies besides confirmation of the progress of a reaction and mass mapping. Examination of the RP-HPLC fractionated fractions by a sequencer and TOF-MS revealed that most of the fragments of each chain of each antibody were recovered.

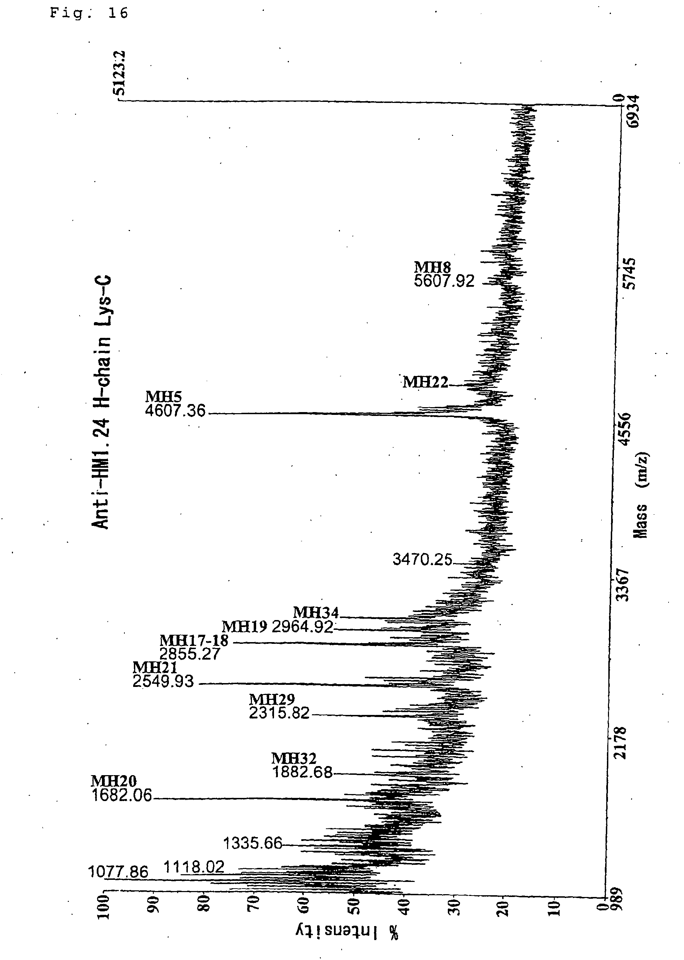

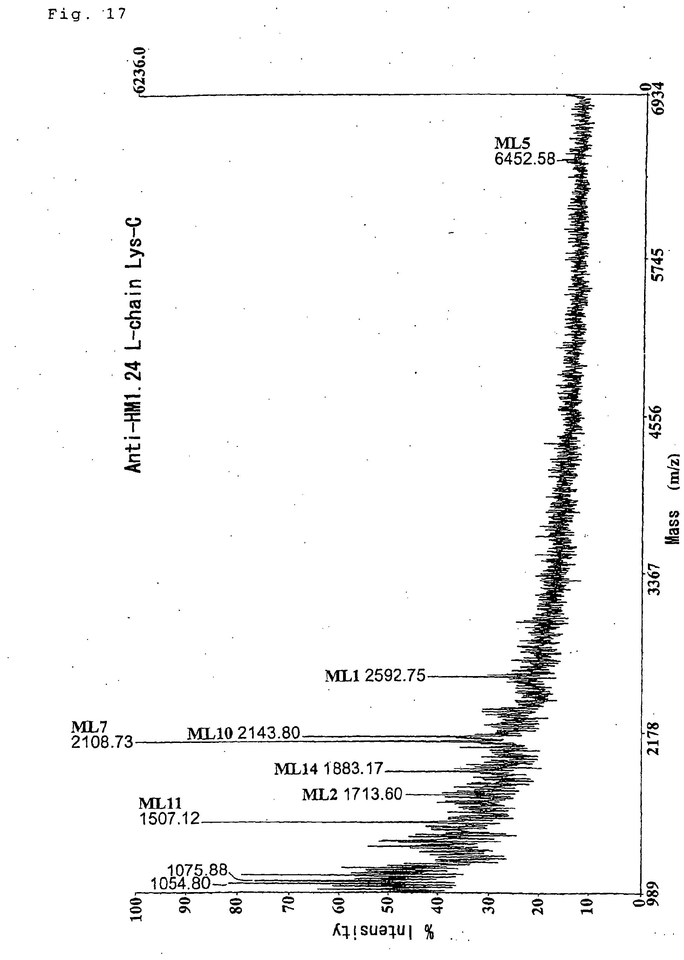

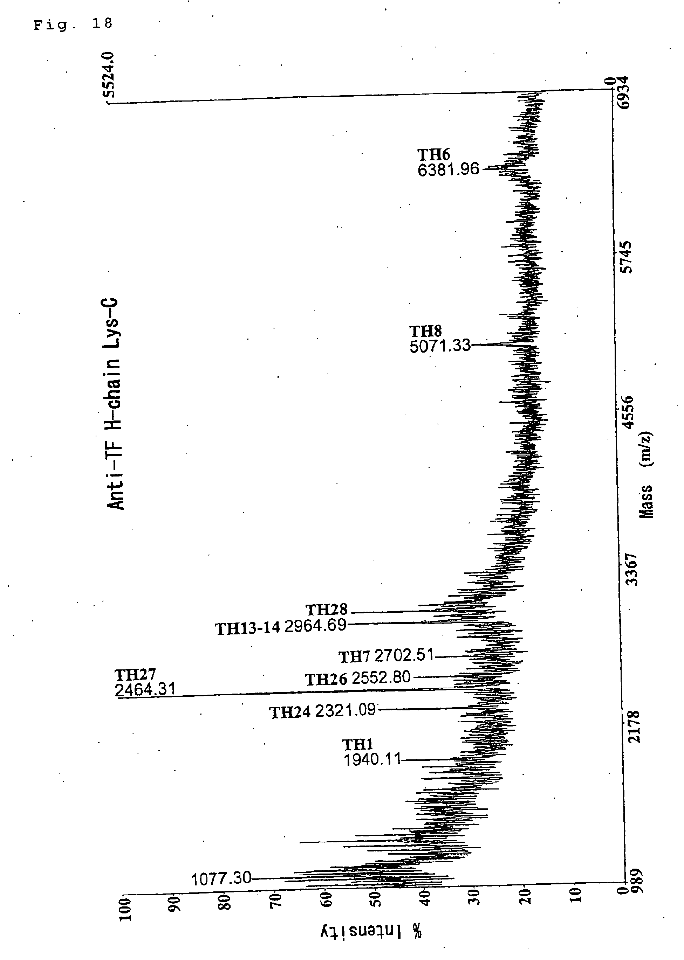

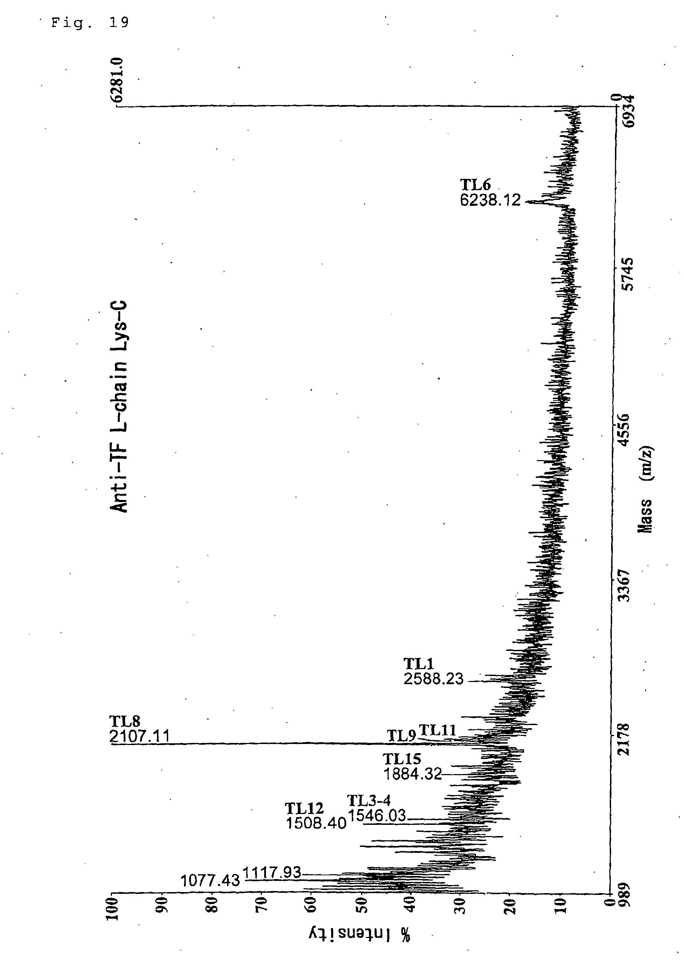

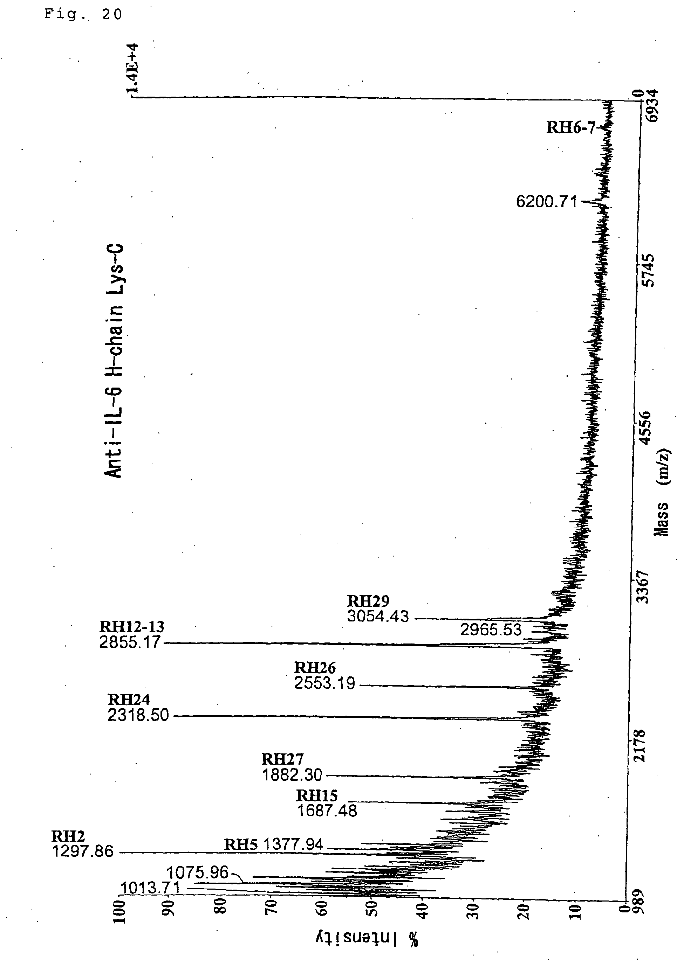

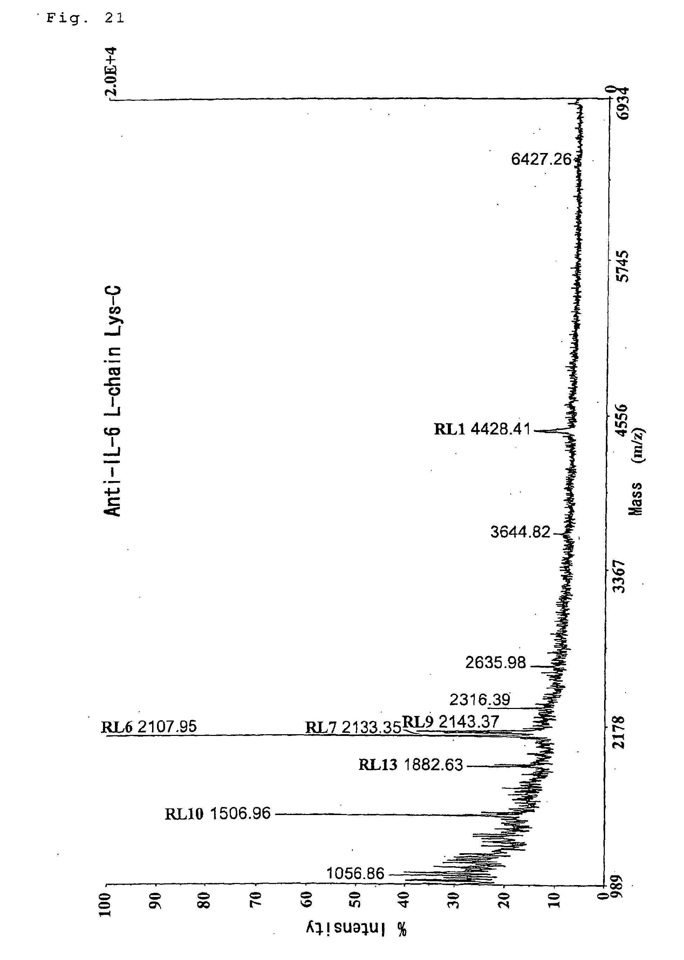

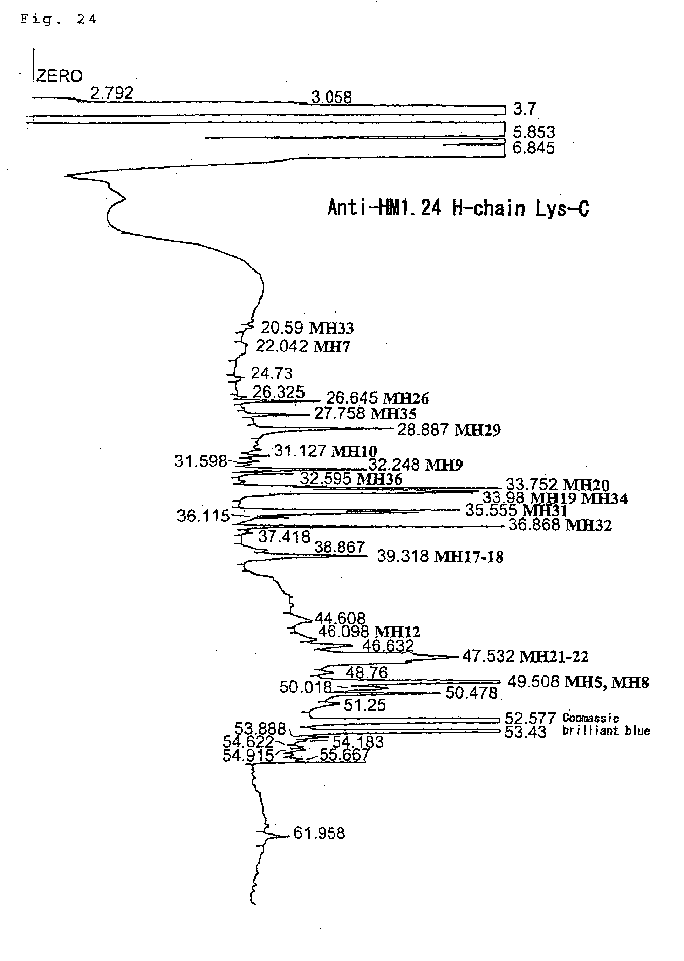

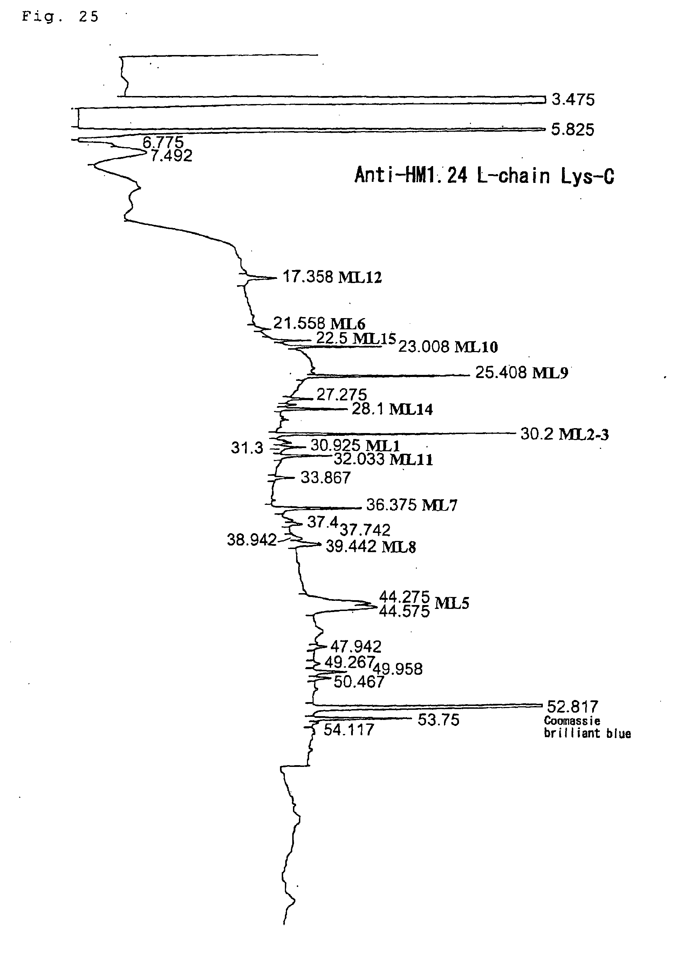

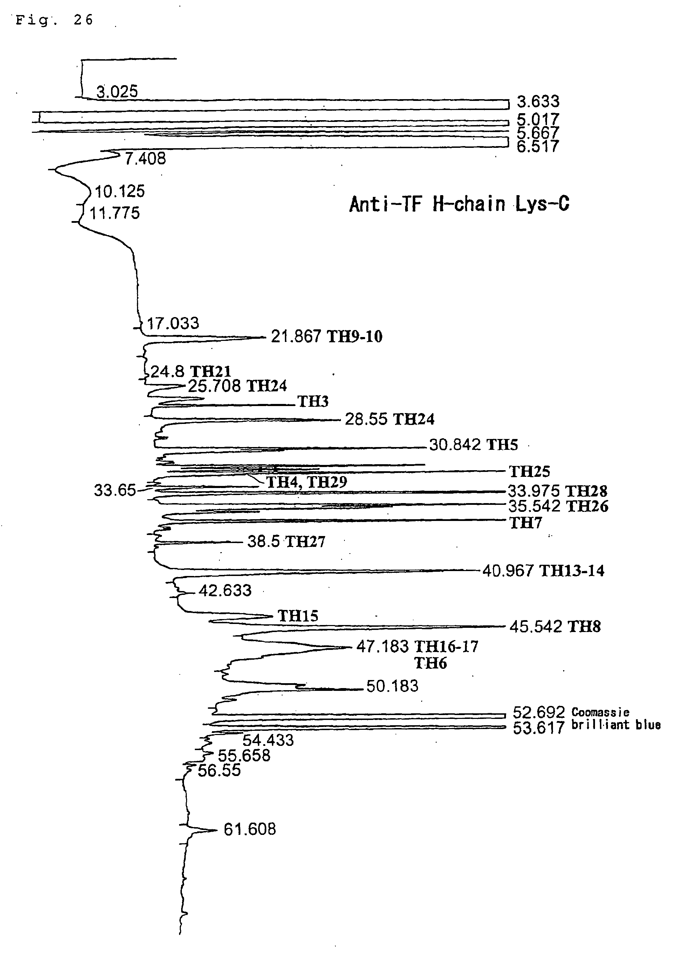

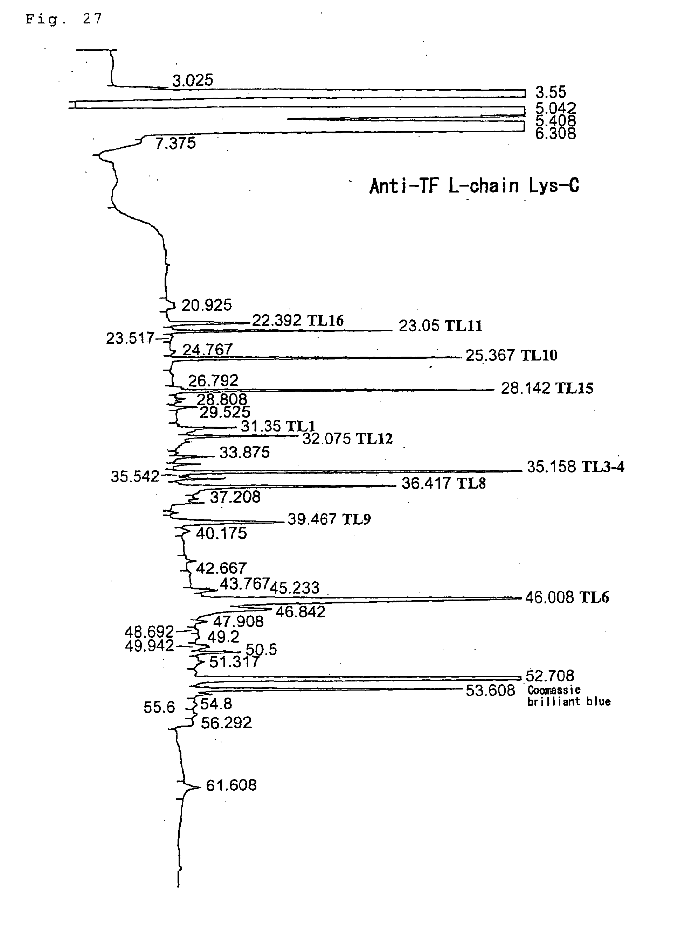

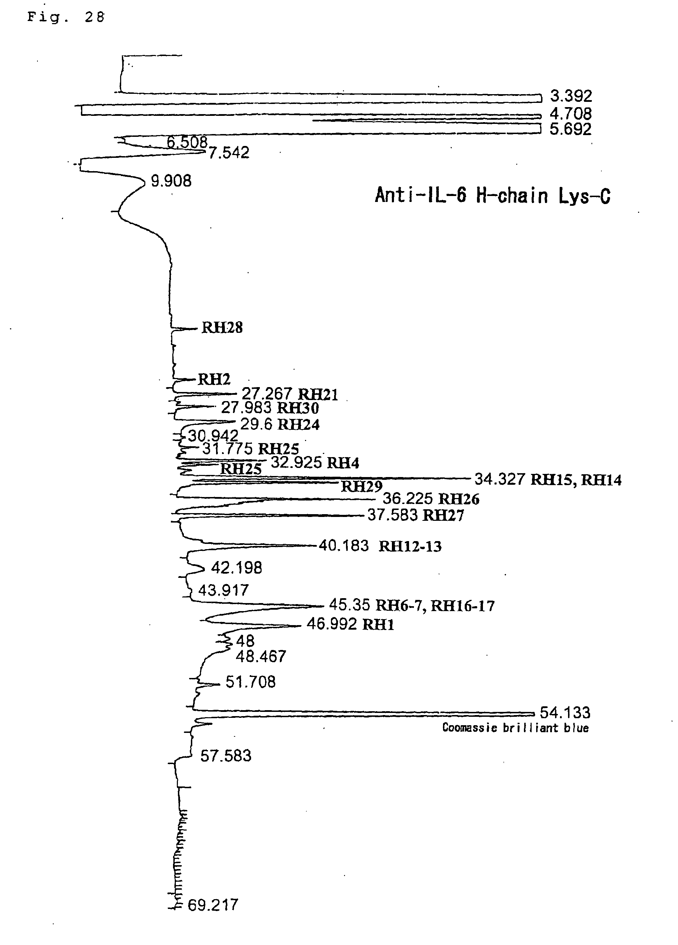

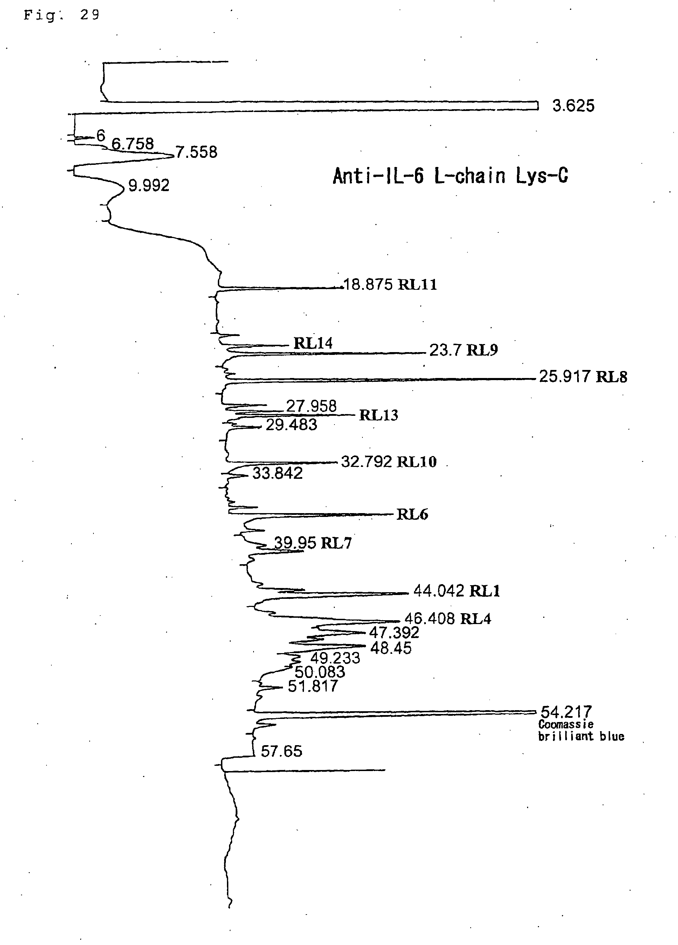

Fragments that are predicted to be generated by the digestion with Lys-C based on the amino acid sequences of the L-chains and H-chains of the above-mentioned humanized anti-HM1.24 antibody, humanized anti-TF antibody, and humanized anti-IL6-R antibody are listed in Tables 7 to 12. The tables show from the left, the fragment number, amino acid number, amino acid sequence of the fragment, and the theoretical mass. Furthermore, in the tables, fragments to which the peaks could be assigned in the TOF-MS spectra (Figs. 16 to 21) were marked with circles (○). Furthermore, fragments in which the assignments could be confirmed by analyzing the fractions by RP-HPLC (Figs. 24 to 29) were marked with stars ( 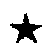 ).

| | L-chain | H-chain |
|---|---|---|
| Humanized anti-HM1.24 antibody | Table 7 | Table 8 |
| Humanized anti-TF antibody | Table 9 | Table 10 |
| Humanized anti-IL-6R antibody | Table 11 | Table 12 |

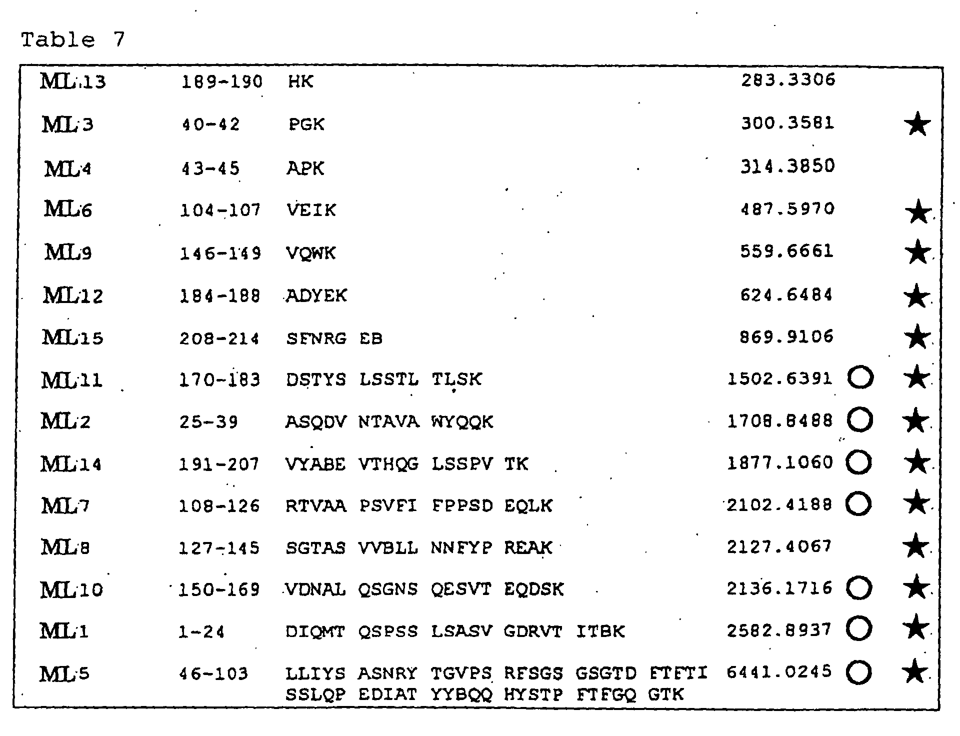

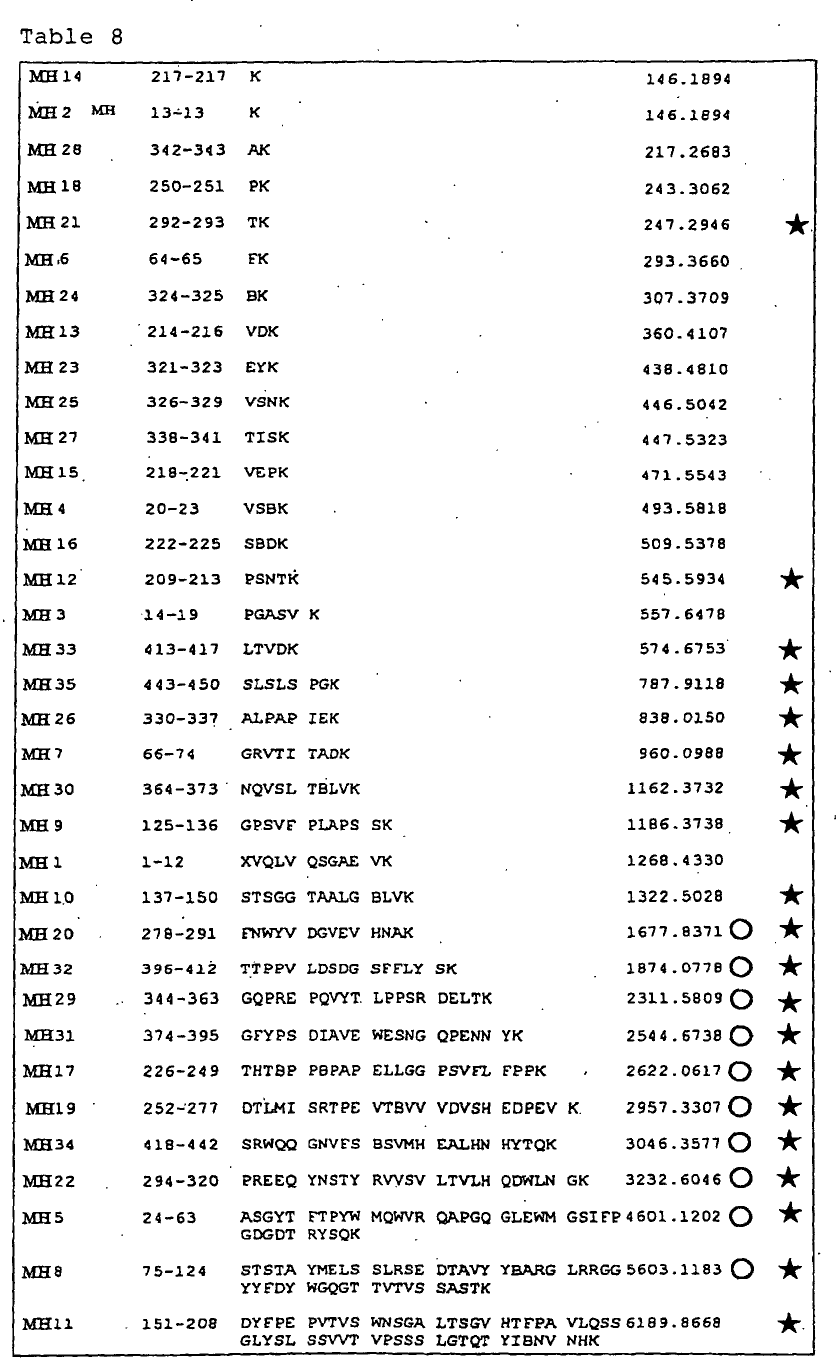

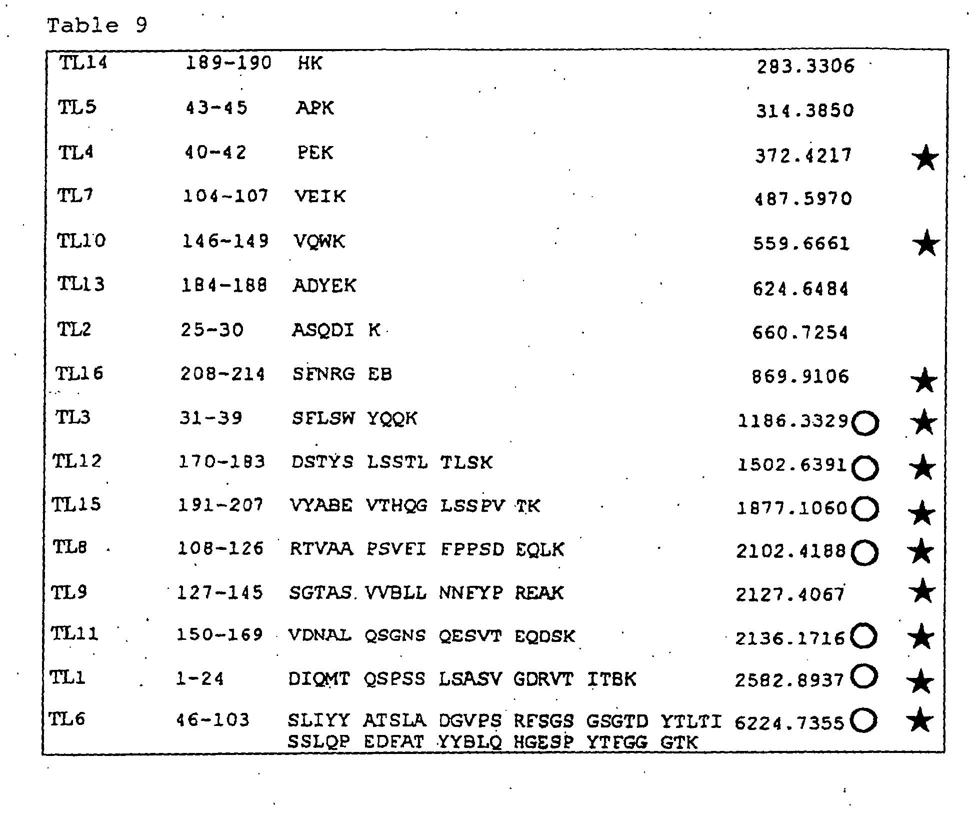

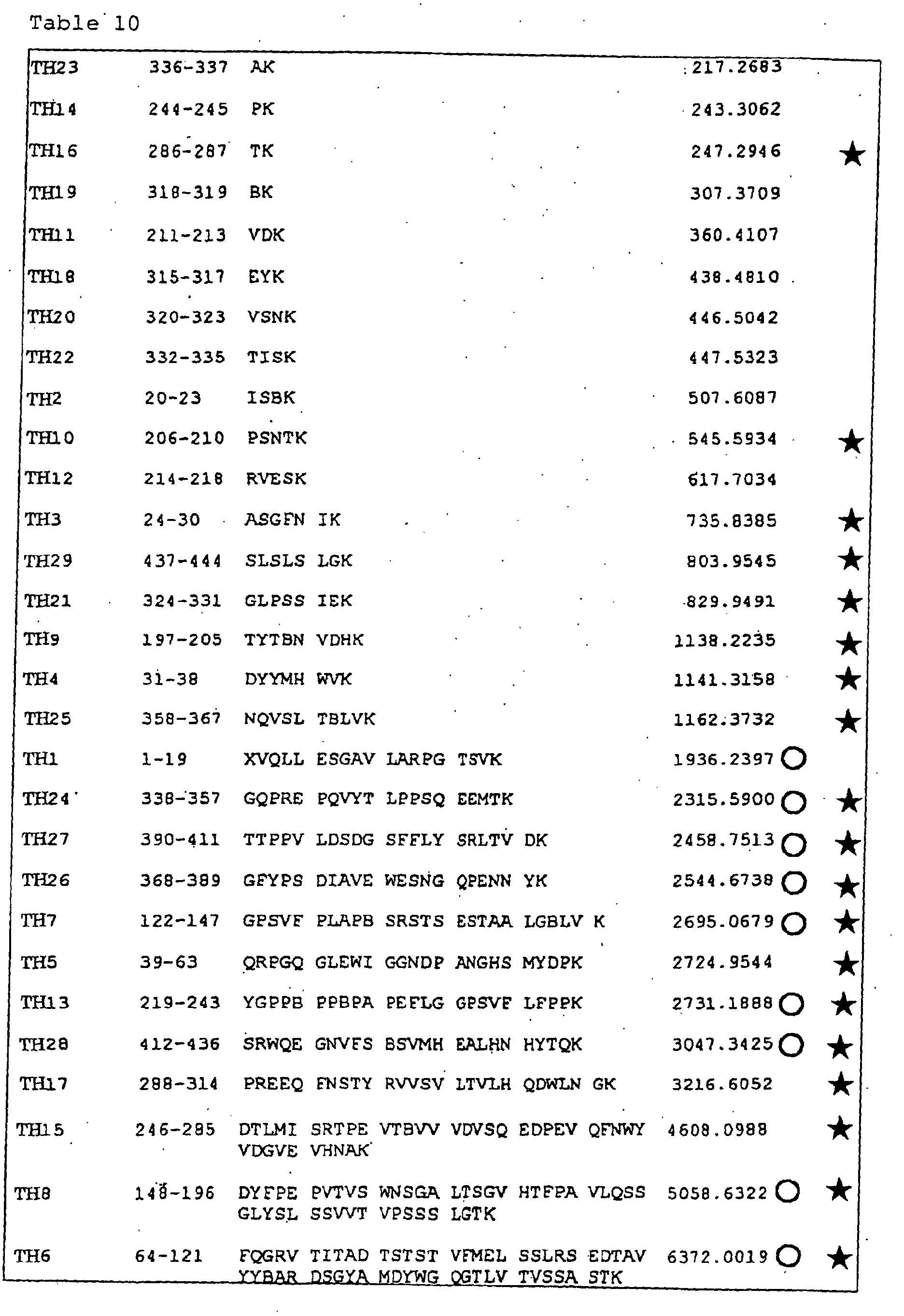

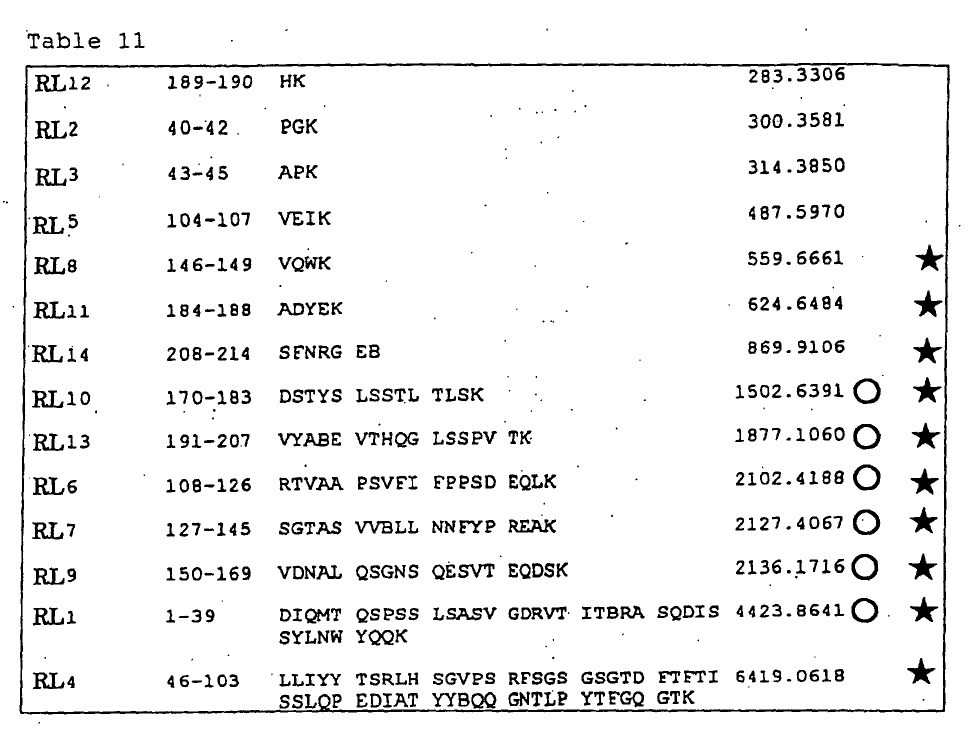

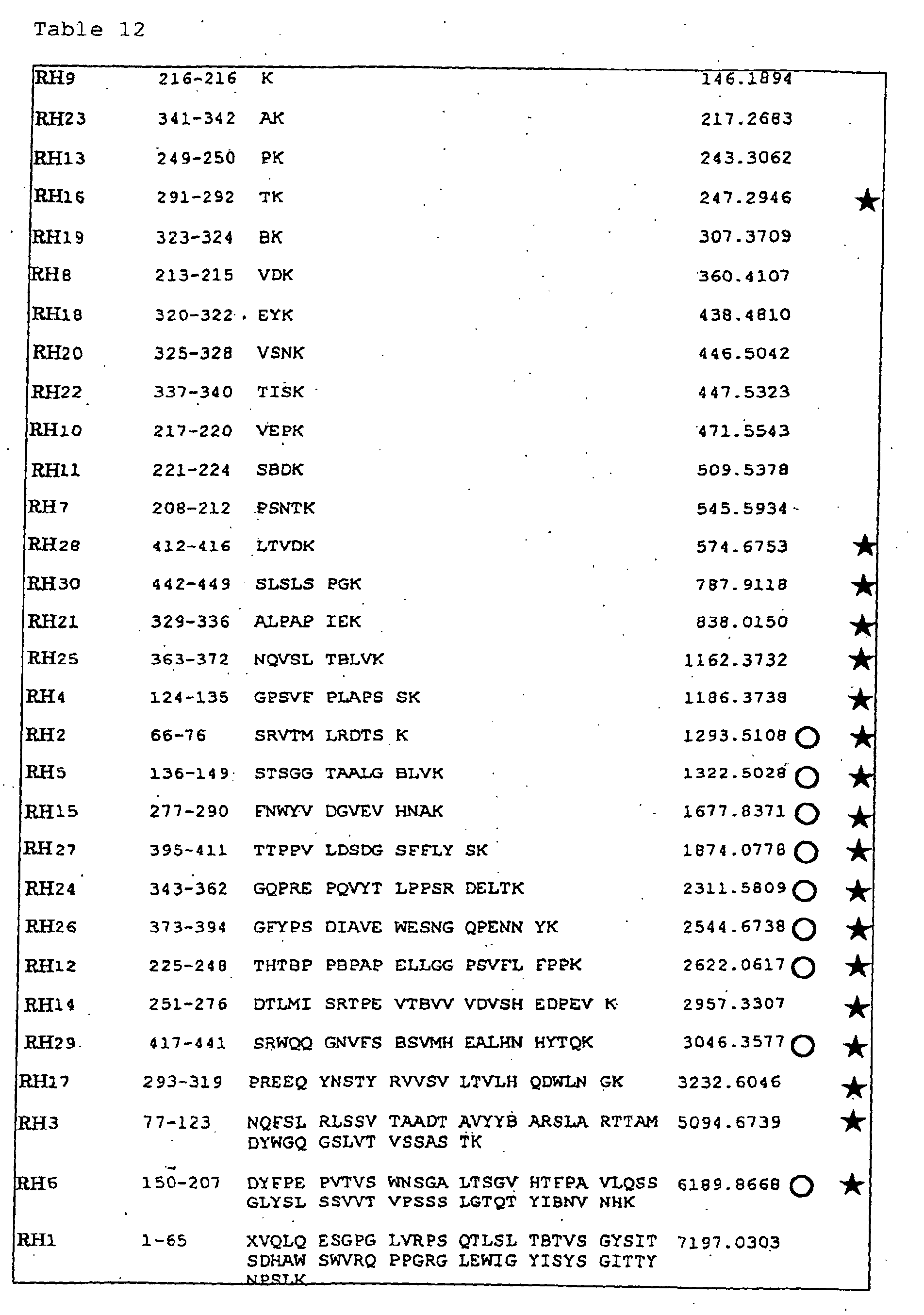

### [Example 4]

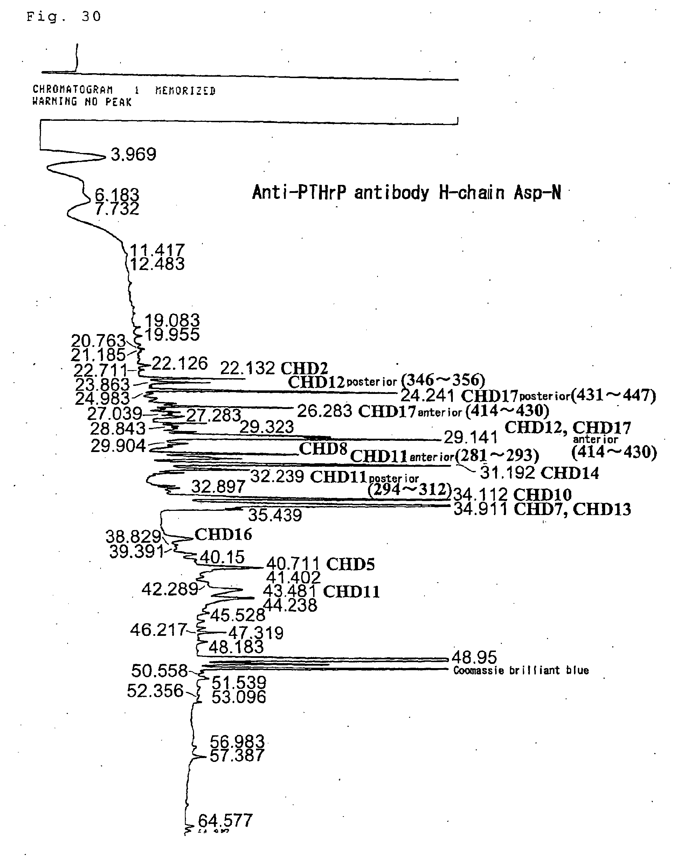

The H-chain of humanized anti-PTHrP antibody was digested with Asp-N under the same conditions as in Example 2, and was analyzed by RP-HPLC. The result of analysis (chromatogram) is shown in Fig. 30.

### Industrial Applicability

According to the present invention, peptide fragments of a fragmented antibody molecule can be analyzed accurately in terms of both the kind and amount. Consequently, peptide maps with a good reproducibility can be prepared, which allows analysis of heterogeneity and evaluation of the quality of the antibody molecule to be more conveniently and more quickly carried out. Furthermore, by comparing the peptide maps obtained according to the present invention, identification of the subclass of structurally unidentified antibodies, and identification of the variable region can be easily carried out.

Additionally, according to the method for analyzing antibody molecules of the present invention, accurate analysis of an antibody molecule can be performed with a small amount of antibody sample. The high reproducibility of the analysis method of the present invention allows analysis with a small amount of sample.

Furthermore, according to the present invention, reproducibility could be also confirmed for the side-reaction products in the digest mixture, indicating that, not only theoretically predicted reaction products but also side-reaction products that are theoretically difficult to predict can be detected with a high reproducibility according to the analysis method of the present invention. This means that peptide maps of the present invention allow more precise and more accurate characterization of antibody molecules.

## Claims

1. A method for structural analysis of an antibody molecule, which comprises the steps of:
a) separating the antibody molecule into H-chains and L-chains;
b) fragmenting the separated H-chains and L-chains; and
c) analyzing the peptide fragments obtained by fragmentation.

2. The method of claim 1, wherein the antibody molecule is separated into H-chains and L-chains by denaturing gel electrophoresis.

3. The method of claim 1, wherein disulfide bonds are cleaved by reduction prior to separating the antibody molecule into H-chains and L-chains.

4. The method of claim 3, wherein the thiol groups formed by reducing the disulfide bonds of the antibody molecule are made irreversible by modification.

5. The method of claim 2, wherein the separated H-chains and L-chains are fragmented in gel.

6. The method of claim 5 which comprises the step of eluting the fragmented peptides from the gel.

7. The method of claim 2, wherein the separated H-chains and L-chains are transferred to a protein adsorption membrane, and then are fragmented on the protein adsorption membrane.

8. The method of claim 1 comprising the step of separating the peptide fragments obtained by fragmentation.

9. The method of claim 8 additionally comprising the step of deblocking peptide fragments whose N-termini are blocked.

10. The method of claim 1, wherein the analysis of peptide fragments is performed by amino acid sequence analysis.

11. The method of claim 1, wherein the analysis of peptide fragments is performed by mass spectrometry.

12. The method of claim 1, wherein the analysis of peptide fragments is performed by amino acid composition analysis.

13. A peptide map which is produced based on results of structural analysis obtainable by the method of claim 1.

14. The method of claim 1 wherein the type of heterogeneity is determined by analyzing peptide fragments involved in heterogenity among the peptide fragments obtained by separation.

15. The method of claim 1, wherein the antibody is an antibody for pharmaceutical use.

16. A method of evaluating the quality of an antibody molecule, which comprises the steps of:
a) separating the antibody molecule into H-chains and L-chains;
b) fragmenting the separated H-chains and L-chains;
c) analyzing the peptide fragments obtained by fragmentation;
d) comparing the obtained analysis result with an analysis result obtained by analyzing a structurally identified antibody; and
e) determining that the two antibodies are the same when there is no difference between the two analysis results, or when the difference is within a specified range.

17. A method for identifying the subclass of'the H-chain and/or the type of the L-chain of a structurally unidentified antibody by analyzing the structures of a structurally identified antibody and a structurally unidentified antibody, respectively, by the method of claim 1, and then comparing the analysis results relating to both constant regions.

18. A method for analyzing the variable region of an antibody molecule, which comprises the steps of:
a) analyzing the structure of the antibody molecule by the method of claim 1; and
b) selecting information on the variable region by removing information on the constant region from the analyzed information of a) based on the analysis result of a structurally identified antibody.

19. The method of claim 18, wherein the analysis method is reverse-phase liquid chromatography, and step b) involves selecting peaks corresponding to the variable region from peaks corresponding to the constant region.
